# EUROPEAN PATENT APPLICATION

(11) **EP 2 270 142 A1**
(43) Date of publication of application: **05.01.2011**
(21) Application number: 09719525.9
(22) Date of filing: 10.03.2009
(51) Int. Cl.: C12N 15/09, C12Q 1/68

(54) **METHOD OF PREPARING DNA FRAGMENT HAVING STICKY END**

(30) Priority: 11.03.2008 JP 2008061678
(71) Applicant: The University of Tokyo, Bunkyo-Ku Tokyo 113-8654 (JP)
(72) Inventor: KOMIYAMA, Makoto, Tokyo 113-8654 (JP); KUZUYA, Akinori, Tokyo 113-8654 (JP); TANAKA, Keita, Tokyo 113-8654 (JP)
(74) Representative: Denison, Christopher Marcus
(86) International application number: PCT/JP2009/054989
(87) International publication number: WO 2009/113709

(57) **Abstract**

The present invention provides a method for preparing a DNA fragment, in which a desired double-stranded DNA fragment having a sticky end is directly and easily obtained from an amplification product (an amplified fragment) after PCR without a restriction enzyme digestion. The method for preparing a DNA fragment having a sticky end of the present invention comprises: (i) a step of performing a PCR reaction using a template DNA and specific primers to obtain an amplified DNA fragment; and (ii) a step of performing a prescribed treatment on the amplified DNA fragment to dissociate a protecting group from the fragment. Herein, the above-mentioned specific primers are composed of a complementary DNA portion consisting of a nucleotide sequence complementarily binding to an amplification target region in a template DNA and a non-complementary DNA portion consisting of a nucleotide sequence that links to the 5' end of the complementary DNA portion but does not complementarily bind to the amplification target sequence, and at least a base corresponding to the 3' end in the nucleotide sequence of the non-complementary DNA portion is modified with a protecting group capable of terminating the progression of DNA replication catalyzed by a DNA polymerase.

SEQUENCE LISTING

<110> THE UNIVERSITY OF TOKYO

<120> Method for producing of DNA fragmnt having a cohesive end

<130> PCT09-0004

<150> JP 2008-061678
<151> 2008-03-11

<160> 15

<170> PatentIn version 3.4

<210> 1
<211> 24
<212> DNA
<213> Artificial

<220>
<223> Synthetic DNA

<220>
<221> modified_base
<222> 4
<223> t is NPP-modified base

<400> 1
agctgtccgg cgtagaggat cgag 24

<210> 2
<211> 17
<212> DNA
<213> Artificial

<220>
<223> Synthetic DNA

<400> 2
ctcgatcctc tacgccg 17

<210> 3
<211> 24
<212> DNA
<213> Artificial

<220>
<223>  Synthetic DNA

<220>
<221>  modified_base
<222>  3
<223>  t is NPP-modified base

<220>
<221>  modified_base
<222>  4
<223>  t is NPP-modified base

<400>  3
aattcaccgt caccctggat gctg                                              24


<210>  4
<211>  24
<212>  DNA
<213>  Artificial

<220>
<223>  Synthetic DNA

<220>
<221>  modified_base
<222>  3
<223>  t is NPP-modified base

<220>
<221>  modified_base
<222>  4
<223>  t is NPP-modified base

<400>  4
aattgtccgg cgtagaggat cgag                                              24


<210>  5
<211>  36
<212>  DNA
<213>  Artificial

<220>
<223>  Synthetic DNA

<220>
<221>  modified_base

<222>  9
<223>  t is NPP-modified base

<220>
<221>  modified_base
<222>  16
<223>  t is NPP-modified base

<400>  5
atcatcagta acccgtcacc gtcaccctgg atgctg                                36


<210>  6
<211>  36
<212>  DNA
<213>  Artificial

<220>
<223>  Synthetic DNA

<220>
<221>  modified_base
<222>  8
<223>  t is NPP-modified base

<220>
<221>  modified_base
<222>  16
<223>  t is NPP-modified base

<400>  6
acgggttact gatgatgtcc ggcgtagagg atcgag                                36


<210>  7
<211>  4361
<212>  DNA
<213>  Artificial

<220>
<223>  Cloning vector pBR322

<400>  7
ttctcatgtt tgacagctta tcatcgataa gctttaatgc ggtagtttat cacagttaaa      60

ttgctaacgc agtcaggcac cgtgtatgaa atctaacaat gcgctcatcg tcatcctcgg     120

caccgtcacc ctggatgctg taggcatagg cttggttatg ccggtactgc cgggcctctt     180

gcgggatatc gtccattccg acagcatcgc cagtcactat ggcgtgctgc tagcgctata 240

tgcgttgatg caatttctat gcgcacccgt tctcggagca ctgtccgacc gctttggccg 300

ccgcccagtc ctgctcgctt cgctacttgg agccactatc gactacgcga tcatggcgac 360

cacacccgtc ctgtggatcc tctacgccgg acgcatcgtg gccggcatca ccggcgccac 420

aggtgcggtt gctggcgcct atatcgccga catcaccgat ggggaagatc gggctcgcca 480

cttcgggctc atgagcgctt gtttcggcgt gggtatggtg gcaggccccg tggccggggg 540

actgttgggc gccatctcct tgcatgcacc attccttgcg gcggcggtgc tcaacggcct 600

caacctacta ctgggctgct tcctaatgca ggagtcgcat aagggagagc gtcgaccgat 660

gcccttgaga gccttcaacc cagtcagctc cttccggtgg gcgcggggca tgactatcgt 720

cgccgcactt atgactgtct tctttatcat gcaactcgta ggacaggtgc cggcagcgct 780

ctgggtcatt ttcggcgagg accgctttcg ctggagcgcg acgatgatcg gcctgtcgct 840

tgcggtattc ggaatcttgc acgccctcgc tcaagccttc gtcactggtc ccgccaccaa 900

acgtttcggc gagaagcagg ccattatcgc cggcatggcg gccgacgcgc tgggctacgt 960

cttgctggcg ttcgcgacgc gaggctggat ggccttcccc attatgattc ttctcgcttc 1020

cggcggcatc gggatgcccg cgttgcaggc catgctgtcc aggcaggtag atgacgacca 1080

tcagggacag cttcaaggat cgctcgcggc tcttaccagc ctaacttcga tcactggacc 1140

gctgatcgtc acggcgattt atgccgcctc ggcgagcaca tggaacgggt tggcatggat 1200

tgtaggcgcc gccctatacc ttgtctgcct ccccgcgttg cgtcgcggtg catggagccg 1260

ggccacctcg acctgaatgg aagccggcgg cacctcgcta acggattcac cactccaaga 1320

attggagcca atcaattctt gcggagaact gtgaatgcgc aaaccaaccc ttggcagaac 1380

atatccatcg cgtccgccat ctccagcagc cgcacgcggc gcatctcggg cagcgttggg 1440

tcctggccac gggtgcgcat gatcgtgctc ctgtcgttga ggacccggct aggctggcgg 1500

ggttgcctta ctggttagca gaatgaatca ccgatacgcg agcgaacgtg aagcgactgc 1560

tgctgcaaaa cgtctgcgac ctgagcaaca acatgaatgg tcttcggttt ccgtgtttcg 1620

taaagtctgg aaacgcggaa gtcagcgccc tgcaccatta tgttccggat ctgcatcgca 1680

ggatgctgct ggctaccctg tggaacacct acatctgtat taacgaagcg ctggcattga 1740

ccctgagtga tttttctctg gtcccgccgc atccataccg ccagttgttt accctcacaa 1800

cgttccagta accgggcatg ttcatcatca gtaacccgta tcgtgagcat cctctctcgt 1860

ttcatcggta tcattacccc catgaacaga aatccccctt acacggaggc atcagtgacc 1920

aaacaggaaa aaaccgccct taacatggcc cgctttatca gaagccagac attaacgctt 1980

ctggagaaac tcaacgagct ggacgcggat gaacaggcag acatctgtga atcgcttcac 2040

gaccacgctg atgagcttta ccgcagctgc ctcgcgcgtt tcggtgatga cggtgaaaac 2100

ctctgacaca tgcagctccc ggagacggtc acagcttgtc tgtaagcgga tgccgggagc 2160

agacaagccc gtcagggcgc gtcagcgggt gttggcgggt gtcggggcgc agccatgacc 2220

cagtcacgta gcgatagcgg agtgtatact ggcttaacta tgcggcatca gagcagattg 2280

tactgagagt gcaccatatg cggtgtgaaa taccgcacag atgcgtaagg agaaaatacc 2340

gcatcaggcg ctcttccgct tcctcgctca ctgactcgct gcgctcggtc gttcggctgc 2400

ggcgagcggt atcagctcac tcaaaggcgg taatacggtt atccacagaa tcaggggata 2460

acgcaggaaa gaacatgtga gcaaaaggcc agcaaaaggc caggaaccgt aaaaaggccg 2520

cgttgctggc gtttttccat aggctccgcc cccctgacga gcatcacaaa aatcgacgct 2580

caagtcagag gtggcgaaac ccgacaggac tataaagata ccaggcgttt ccccctggaa 2640

gctccctcgt gcgctctcct gttccgaccc tgccgcttac cggatacctg tccgcctttc 2700

tcccttcggg aagcgtggcg ctttctcata gctcacgctg taggtatctc agttcggtgt 2760

aggtcgttcg ctccaagctg ggctgtgtgc acgaaccccc cgttcagccc gaccgctgcg 2820

ccttatccgg taactatcgt cttgagtcca acccggtaag acacgactta tcgccactgg 2880

cagcagccac tggtaacagg attagcagag cgaggtatgt aggcggtgct acagagttct 2940

tgaagtggtg gcctaactac ggctacacta gaaggacagt atttggtatc tgcgctctgc 3000

tgaagccagt taccttcgga aaaagagttg gtagctcttg atccggcaaa caaaccaccg 3060

ctggtagcgg tggttttttt gtttgcaagc agcagattac gcgcagaaaa aaaggatctc 3120

aagaagatcc tttgatcttt tctacggggt ctgacgctca gtggaacgaa aactcacgtt 3180

aagggatttt ggtcatgaga ttatcaaaaa ggatcttcac ctagatcctt ttaaattaaa 3240

aatgaagttt taaatcaatc taaagtatat atgagtaaac ttggtctgac agttaccaat 3300

gcttaatcag tgaggcacct atctcagcga tctgtctatt tcgttcatcc atagttgcct 3360

gactccccgt cgtgtagata actacgatac gggagggctt accatctggc cccagtgctg 3420

caatgatacc gcgagaccca cgctcaccgg ctccagattt atcagcaata aaccagccag 3480

ccggaagggc cgagcgcaga agtggtcctg caactttatc cgcctccatc cagtctatta 3540

attgttgccg ggaagctaga gtaagtagtt cgccagttaa tagtttgcgc aacgttgttg 3600

ccattgctgc aggcatcgtg gtgtcacgct cgtcgtttgg tatggcttca ttcagctccg 3660

gttcccaacg atcaaggcga gttacatgat cccccatgtt gtgcaaaaaa gcggttagct 3720

ccttcggtcc tccgatcgtt gtcagaagta agttggccgc agtgttatca ctcatggtta 3780

tggcagcact gcataattct cttactgtca tgccatccgt aagatgcttt tctgtgactg 3840

gtgagtactc aaccaagtca ttctgagaat agtgtatgcg gcgaccgagt tgctcttgcc 3900

cggcgtcaac acgggataat accgcgccac atagcagaac tttaaaagtg ctcatcattg 3960

gaaaacgttc ttcggggcga aaactctcaa ggatcttacc gctgttgaga tccagttcga 4020

tgtaacccac tcgtgcaccc aactgatctt cagcatcttt tactttcacc agcgtttctg 4080

ggtgagcaaa aacaggaagg caaaatgccg caaaaaaggg aataagggcg acacggaaat 4140

gttgaatact catactcttc ctttttcaat attattgaag catttatcag ggttattgtc 4200

tcatgagcgg atacatattt gaatgtattt agaaaaataa acaaataggg gttccgcgca 4260

catttccccg aaaagtgcca cctgacgtct aagaaaccat tattatcatg acattaacct 4320

ataaaaatag gcgtatcacg aggccctttc gtcttcaaga a 4361

<210> 8
<211> 2501
<212> DNA
<213> Artificial

<220>

<223>  Cloning vector pBR322

<400>  8
tcctctctcg tttcatcggt atcattaccc ccatgaacag aaatccccct tacacggagg      60

catcagtgac caaacaggaa aaaaccgccc ttaacatggc ccgctttatc agaagccaga     120

cattaacgct tctggagaaa ctcaacgagc tggacgcgga tgaacaggca gacatctgtg     180

aatcgcttca cgaccacgct gatgagcttt accgcagctg cctcgcgcgt ttcggtgatg     240

acggtgaaaa cctctgacac atgcagctcc cggagacggt cacagcttgt ctgtaagcgg     300

atgccgggag cagacaagcc cgtcagggcg cgtcagcggg tgttggcggg tgtcggggcg     360

cagccatgac ccagtcacgt agcgatagcg gagtgtatac tggcttaact atgcggcatc     420

agagcagatt gtactgagag tgcaccatat gcggtgtgaa ataccgcaca gatgcgtaag     480

gagaaaatac cgcatcaggc gctcttccgc ttcctcgctc actgactcgc tgcgctcggt     540

cgttcggctg cggcgagcgg tatcagctca ctcaaaggcg gtaatacggt tatccacaga     600

atcaggggat aacgcaggaa agaacatgtg agcaaaaggc cagcaaaagg ccaggaaccg     660

taaaaaggcc gcgttgctgg cgtttttcca taggctccgc ccccctgacg agcatcacaa     720

aaatcgacgc tcaagtcaga ggtggcgaaa cccgacagga ctataaagat accaggcgtt     780

tccccctgga agctccctcg tgcgctctcc tgttccgacc ctgccgctta ccggatacct     840

gtccgccttt ctcccttcgg gaagcgtggc gctttctcat agctcacgct gtaggtatct     900

cagttcggtg taggtcgttc gctccaagct gggctgtgtg cacgaacccc ccgttcagcc     960

cgaccgctgc gccttatccg gtaactatcg tcttgagtcc aacccggtaa gacacgactt    1020

atcgccactg gcagcagcca ctggtaacag gattagcaga gcgaggtatg taggcggtgc    1080

tacagagttc ttgaagtggt ggcctaacta cggctacact agaaggacag tatttggtat    1140

ctgcgctctg ctgaagccag ttaccttcgg aaaaagagtt ggtagctctt gatccggcaa    1200

acaaaccacc gctggtagcg gtggtttttt tgtttgcaag cagcagatta cgcgcagaaa    1260

aaaaggatct caagaagatc ctttgatctt ttctacgggg tctgacgctc agtggaacga    1320

aaactcacgt taagggattt tggtcatgag attatcaaaa aggatcttca cctagatcct    1380

tttaaattaa aaatgaagtt ttaaatcaat ctaaagtata tatgagtaaa cttggtctga 1440

cagttaccaa tgcttaatca gtgaggcacc tatctcagcg atctgtctat ttcgttcatc 1500

catagttgcc tgactccccg tcgtgtagat aactacgata cgggagggct taccatctgg 1560

ccccagtgct gcaatgatac cgcgagaccc acgctcaccg gctccagatt tatcagcaat 1620

aaaccagcca gccggaaggg ccgagcgcag aagtggtcct gcaactttat ccgcctccat 1680

ccagtctatt aattgttgcc gggaagctag agtaagtagt tcgccagtta atagtttgcg 1740

caacgttgtt gccattgctg caggcatcgt ggtgtcacgc tcgtcgtttg gtatggcttc 1800

attcagctcc ggttcccaac gatcaaggcg agttacatga tcccccatgt tgtgcaaaaa 1860

agcggttagc tccttcggtc ctccgatcgt tgtcagaagt aagttggccg cagtgttatc 1920

actcatggtt atggcagcac tgcataattc tcttactgtc atgccatccg taagatgctt 1980

ttctgtgact ggtgagtact caaccaagtc attctgagaa tagtgtatgc ggcgaccgag 2040

ttgctcttgc ccggcgtcaa cacgggataa taccgcgcca catagcagaa ctttaaaagt 2100

gctcatcatt ggaaaacgtt cttcggggcg aaaactctca aggatcttac cgctgttgag 2160

atccagttcg atgtaaccca ctcgtgcacc caactgatct tcagcatctt ttactttcac 2220

cagcgtttct gggtgagcaa aaacaggaag gcaaaatgcc gcaaaaaagg gaataagggc 2280

gacacggaaa tgttgaatac tcatactctt cctttttcaa tattattgaa gcatttatca 2340

gggttattgt ctcatgagcg gatacatatt tgaatgtatt tagaaaaata aacaaatagg 2400

ggttccgcgc acatttcccc gaaaagtgcc acctgacgtc taagaaacca ttattatcat 2460

gacattaacc tataaaaata ggcgtatcac gaggcccttt c 2501

<210> 9
<211> 5115
<212> DNA
<213> Artificial

<220>
<223> Cloning vector pQBI T7-GFP

<400> 9
ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc cggatatagt tcctcctttc agcaaaaaac ccctcaagac ccgtttagag 240

gccccaaggg gttatgctag ttattgctca gcggtggcag cagccaactc agcttccttt 300

cgggctttgt tagcagccgg atcctcagtt gtacagttca tccatgccat gtgtaatccc 360

agcagctgtt acaaactcaa gaaggaccat gtggtctctc ttttcgttgg gatctttcga 420

aagggcagat tgtgtggaca ggtaatggtt gtctggtaaa aggacagggc catcgccaat 480

tggagtattt tgttgataat ggtctgctag ttgaacgctt ccatcttcaa tgttgtggcg 540

ggtcttgaag ttcactttga ttccattctt ttgtttgtct gccatgatgt atacattgtg 600

tgagttatag ttgtattcca atttgtgtcc cagaatgttg ccatcttcct tgaagtcaat 660

accttttaac tcgattctat taacaagggt atcaccttca aacttgactt cagcacgtgt 720

cttgtagttg ccgtcatctt tgaagaagat ggtcctttcc tgtacataac cttcgggcat 780

ggcactcttg aaaaagtcat gccgtttcat atgatccggg tatcttgaaa agcattgaac 840

accatagcac agagtagtga ctagtgttgg ccatggaaca ggcagtttgc cagtagtgca 900

gatgaacttc agggtaagtt ttccgtatgt tgcatcacct tcaccctctc cactgacaga 960

gaacttgtgg ccgttaacat caccatctaa ttcaacaaga attgggacaa ctccagtgaa 1020

gagttcttct cctttgctag ccatatgtat atctccttct taaagttaaa caaaattatt 1080

tctagagggg aattgttatc cgctcacaat tcccctatag tgagtcgtat taatttcgcg 1140

ggatcgagat ctcgatcctc tacgccggac gcatcgtggc cggcatcacc ggcgccacag 1200

gtgcggttgc tggcgcctat atcgccgaca tcaccgatgg ggaagatcgg gctcgccact 1260

tcgggctcat gagcgcttgt ttcggcgtgg gtatggtggc aggccccgtg gccgggggac 1320

tgttgggcgc catctccttg catgcaccat tccttgcggc ggcggtgctc aacggcctca 1380

acctactact gggctgcttc ctaatgcagg agtcgcataa gggagagcgt cgagatcccg 1440

gacaccatcg aatggcgcaa aacctttcgc ggtatggcat gatagcgccc ggaagagagt 1500

caattcaggg tggtgaatgt gaaaccagta acgttatacg atgtcgcaga gtatgccggt 1560

gtctcttatc agaccgtttc ccgcgtggtg aaccaggcca gccacgtttc tgcgaaaacg 1620

cgggaaaaag tggaagcggc gatggcggag ctgaattaca ttcccaaccg cgtggcacaa 1680

caactggcgg gcaaacagtc gttgctgatt ggcgttgcca cctccagtct ggccctgcac 1740

gcgccgtcgc aaattgtcgc ggcgattaaa tctcgcgccg atcaactggg tgccagcgtg 1800

gtggtgtcga tggtagaacg aagcggcgtc gaagcctgta aagcggcggt gcacaatctt 1860

ctcgcgcaac gcgtcagtgg gctgatcatt aactatccgc tggatgacca ggatgccatt 1920

gctgtggaag ctgcctgcac taatgttccg gcgttatttc ttgatgtctc tgaccagaca 1980

cccatcaaca gtattatttt ctcccatgaa gacggtacgc gactgggcgt ggagcatctg 2040

gtcgcattgg gtcaccagca aatcgcgctg ttagcgggcc cattaagttc tgtctcggcg 2100

cgtctgcgtc tggctggctg gcataaatat ctcactcgca atcaaattca gccgatagcg 2160

gaacgggaag gcgactggag tgccatgtcc ggttttcaac aaaccatgca aatgctgaat 2220

gagggcatcg ttcccactgc gatgctggtt gccaacgatc agatggcgct gggcgcaatg 2280

cgcgccatta ccgagtccgg gctgcgcgtt ggtgcggata tctcggtagt gggatacgac 2340

gataccgaag acagctcatg ttatatcccg ccgttaacca ccatcaaaca ggattttcgc 2400

ctgctggggc aaaccagcgt ggaccgcttg ctgcaactct ctcagggcca ggcggtgaag 2460

ggcaatcagc tgttgcccgt ctcactggtg aaaagaaaaa ccaccctggc gcccaatacg 2520

caaaccgcct ctccccgcgc gttggccgat tcattaatgc agctggcacg acaggtttcc 2580

cgactggaaa gcgggcagtg agcgcaacgc aattaatgta agttagctca ctcattaggc 2640

accgggatct cgaccgatgc ccttgagagc cttcaaccca gtcagctcct tccggtgggc 2700

gcggggcatg actatcgtcg ccgcacttat gactgtcttc tttatcatgc aactcgtagg 2760

acaggtgccg gcagcgctct gggtcatttt cggcgaggac cgctttcgct ggagcgcgac 2820

gatgatcggc ctgtcgcttg cggtattcgg aatcttgcac gccctcgctc aagccttcgt 2880

cactggtccc gccaccaaac gtttcggcga gaagcaggcc attatcgccg gcatggcggc 2940

cgacgcgctg ggctacgtct tgctggcgtt cgcagtcacg tagcgatagc ggagtgtata 3000

ctggcttaac tatgcggcat cagagcagat tgtactgaga gtgcaccata tatgcggtgt 3060

gaaataccgc acagatgcgt aaggagaaaa taccgcatca ggcgctcttc cgcttcctcg 3120

ctcactgact cgctgcgctc ggtcgttcgg ctgcggcgag cggtatcagc tcactcaaag 3180

gcggtaatac ggttatccac agaatcaggg gataacgcag gaaagaacat gtgagcaaaa 3240

ggccagcaaa aggccaggaa ccgtaaaaag gccgcgttgc tggcgttttt ccataggctc 3300

cgcccccctg acgagcatca caaaaatcga cgctcaagtc agaggtggcg aaacccgaca 3360

ggactataaa gataccaggc gtttccccct ggaagctccc tcgtgcgctc tcctgttccg 3420

accctgccgc ttaccggata cctgtccgcc tttctccctt cgggaagcgt ggcgctttct 3480

catagctcac gctgtaggta tctcagttcg gtgtaggtcg ttcgctccaa gctgggctgt 3540

gtgcacgaac cccccgttca gcccgaccgc tgcgccttat ccggtaacta tcgtcttgag 3600

tccaacccgg taagacacga cttatcgcca ctggcagcag ccactggtaa caggattagc 3660

agagcgaggt atgtaggcgg tgctacagag ttcttgaagt ggtggcctaa ctacggctac 3720

actagaagga cagtatttgg tatctgcgct ctgctgaagc cagttacctt cggaaaaaga 3780

gttggtagct cttgatccgg caaacaaacc accgctggta gcggtggttt ttttgtttgc 3840

aagcagcaga ttacgcgcag aaaaaaagga tctcaagaag atcctttgat cttttctacg 3900

gggtctgacg ctcagtggaa cgaaaactca cgttaaggga ttttggtcat gagattatca 3960

aaaaggatct tcacctagat ccttttaaat taaaaatgaa gttttaaatc aatctaaagt 4020

atatatgagt aaacttggtc tgacagttac caatgcttaa tcagtgaggc acctatctca 4080

gcgatctgtc tatttcgttc atccatagtt gcctgactcc ccgtcgtgta gataactacg 4140

atacgggagg gcttaccatc tggccccagt gctgcaatga taccgcgaga cccacgctca 4200

ccggctccag atttatcagc aataaaccag ccagccggaa gggccgagcg cagaagtggt 4260

cctgcaactt tatccgcctc catccagtct attaattgtt gccgggaagc tagagtaagt 4320

agttcgccag ttaatagttt gcgcaacgtt gttgccattg ctgcaggcat cgtggtgtca 4380

cgctcgtcgt ttggtatggc ttcattcagc tccggttccc aacgatcaag gcgagttaca 4440

tgatccccca tgttgtgcaa aaaagcggtt agctccttcg gtcctccgat cgttgtcaga 4500

agtaagttgg ccgcagtgtt atcactcatg gttatggcag cactgcataa ttctcttact 4560

gtcatgccat ccgtaagatg cttttctgtg actggtgagt actcaaccaa gtcattctga 4620

gaatagtgta tgcggcgacc gagttgctct tgcccggcgt caacacggga taataccgcg 4680

ccacatagca gaactttaaa agtgctcatc attggaaaac gttcttcggg gcgaaaactc 4740

tcaaggatct taccgctgtt gagatccagt tcgatgtaac ccactcgtgc acccaactga 4800

tcttcagcat cttttacttt caccagcgtt tctgggtgag caaaaacagg aaggcaaaat 4860

gccgcaaaaa agggaataag ggcgacacgg aaatgttgaa tactcatact cttccttttt 4920

caatattatt gaagcattta tcagggttat tgtctcatga gcggatacat atttgaatgt 4980

atttagaaaa ataaacaaat aggggttccg cgcacatttc cccgaaaagt gccacctgac 5040

gtctaagaaa ccattattat catgacatta acctataaaa ataggcgtat cacgaggccc 5100

tttcgtcttc aagaa 5115

<210> 10
<211> 1031
<212> DNA
<213> Artificial

<220>
<223> Cloning vector pQBI T7-GFP

<400> 10
ctggatgctg taggcatagg cttggttatg ccggtactgc cgggcctctt gcgggatatc 60

cggatatagt tcctcctttc agcaaaaaac ccctcaagac ccgtttagag gccccaaggg 120

gttatgctag ttattgctca gcggtggcag cagccaactc agcttccttt cgggctttgt 180

tagcagccgg atcctcagtt gtacagttca tccatgccat gtgtaatccc agcagctgtt 240

acaaactcaa gaaggaccat gtggtctctc ttttcgttgg gatctttcga aagggcagat 300

tgtgtggaca ggtaatggtt gtctggtaaa aggacagggc catcgccaat tggagtattt 360

tgttgataat ggtctgctag ttgaacgctt ccatcttcaa tgttgtggcg ggtcttgaag 420

ttcactttga ttccattctt ttgtttgtct gccatgatgt atacattgtg tgagttatag 480

ttgtattcca atttgtgtcc cagaatgttg ccatcttcct tgaagtcaat accttttaac 540

tcgattctat taacaagggt atcaccttca aacttgactt cagcacgtgt cttgtagttg 600

ccgtcatctt tgaagaagat ggtcctttcc tgtacataac cttcgggcat ggcactcttg 660

aaaaagtcat gccgtttcat atgatccggg tatcttgaaa agcattgaac accatagcac 720

agagtagtga ctagtgttgg ccatggaaca ggcagtttgc cagtagtgca gatgaacttc 780

agggtaagtt ttccgtatgt tgcatcacct tcaccctctc cactgacaga gaacttgtgg 840

ccgttaacat caccatctaa ttcaacaaga attgggacaa ctccagtgaa gagttcttct 900

cctttgctag ccatatgtat atctccttct taaagttaaa caaaattatt tctagagggg 960

aattgttatc cgctcacaat tcccctatag tgagtcgtat taatttcgcg ggatcgagat 1020

ctcgatcctc t 1031

<210> 11
<211> 33
<212> DNA
<213> Artificial

<220>
<223> Synthetic DNA

<400> 11
accgccacct ccgttgtaca gttcatccat gcc 33

<210> 12
<211> 34
<212> DNA
<213> Artificial

<220>
<223> Synthetic DNA

<400> 12
aggatccggc tgctaacaaa gcccgaaagg aagc 34

<210> 13
<211> 34
<212> DNA

<213>  Artificial

<220>
<223>  Synthetic DNA

<400>  13
agccggatcc tcagttgtac agttcatcca tgcc                                34


<210>  14
<211>  36
<212>  DNA
<213>  Artificial

<220>
<223>  Synthetic DNA

<400>  14
aggtggcggt ggcaaaggag aagaactctt cactgg                              36


<210>  15
<211>  701
<212>  DNA
<213>  Artificial

<220>
<223>  Cloning vector pQBI 67 (BFP gene)

<400>  15
agttcatcca tgccatgtgt aatcccagca gctgttacaa actcaagaag gaccatgtgg      60

tctctctttt cgttgggatc tttcgaaagg gcagattgtg tggacaggta atggttgtct     120

ggtaaaagga cagggccatc gccaattgga gtattttgtt gataatggtc tgctagttga     180

acgcttccat cttcaatgtt gtggcggatc ttgaagttcg ctttgattcc attcttttgt     240

ttgtctgcca tgatgtatac attgtgtgag ttatagttgt attccaattt gtgtccaaga     300

atgtttccat cttctttaaa atcaatacct tttaactcga ttctattaac aagggtatca     360

ccttcaaact tgacttcagc acgtgtcttg tagttgccgt catctttgaa gaagatggtc     420

ctttcctgta cataaccttc gggcatggca ctcttgaaaa agtcatgccg tttcatatga     480

tccgggtatc ttgaaaagca ttgaacacca tgagagagag tagtgactag tgttggccat     540

ggaacaggca gtttgccagt agtgcagatg aacttcaggg taagttttcc gtatgttgca     600

tcaccttcac cctctccact gacagagaac ttgtggccgt taacatcacc atctaattca 660

acaagaattg ggacaactcc agtgaagagt tcttctcctt t 701

SEQUENCE LISTING

<110> THE UNIVERSITY OF TOKYO

<120> Method for producing of DNA fragmnt having a cohesive end

<130> PCT09-0004

<150> JP 2008-061678
<151> 2008-03-11

<160> 15

<170> PatentIn version 3.4

<210> 1
<211> 24
<212> DNA
<213> Artificial

<220>
<223> Synthetic DNA

<220>
<221> modified_base
<222> 4
<223> t is NPP-modified base

<400> 1
agctgtccgg cgtagaggat cgag 24

<210> 2
<211> 17
<212> DNA
<213> Artificial

<220>
<223>   Synthetic DNA

<400>   2
ctcgatcctc tacgccg                                                    17

<210>   3
<211>   24
<212>   DNA
<213>   Artificial

<220>
<223>   Synthetic DNA

<220>
<221>   modified_base
<222>   3
<223>   t is NPP-modified base

<220>
<221>   modified_base
<222>   4
<223>   t is NPP-modified base

<400>   3
aattcaccgt caccctggat gctg                                            24

<210>   4
<211>   24
<212>   DNA
<213>   Artificial

<220>
<223>   Synthetic DNA

<220>
<221> modified_base
<222> 3
<223> t is NPP-modified base

<220>
<221> modified_base
<222> 4
<223> t is NPP-modified base

<400> 4
aattgtccgg cgtagaggat cgag 24

<210> 5
<211> 36
<212> DNA
<213> Artificial

<220>
<223> Synthetic DNA

<220>
<221> modified_base
<222> 9
<223> t is NPP-modified base

<220>
<221> modified_base
<222> 16
<223> t is NPP-modified base

<400> 5
atcatcagta acccgtcacc gtcaccctgg atgctg 36

<210> 6

<211>  36
<212>  DNA
<213>  Artificial

<220>
<223>  Synthetic DNA

<220>
<221>  modified_base
<222>  8
<223>  t is NPP-modified base

<220>
<221>  modified_base
<222>  16
<223>  t is NPP-modified base

<400>  6
acgggttact gatgatgtcc ggcgtagagg atcgag                                    36


<210>  7
<211>  4361
<212>  DNA
<213>  Artificial

<220>
<223>  Cloning vector pBR322

<400>  7
ttctcatgtt tgacagctta tcatcgataa gctttaatgc ggtagtttat cacagttaaa        60

ttgctaacgc agtcaggcac cgtgtatgaa atctaacaat gcgctcatcg tcatcctcgg        120

caccgtcacc ctggatgctg taggcatagg cttggttatg ccggtactgc cgggcctctt        180

gcgggatatc gtccattccg acagcatcgc cagtcactat ggcgtgctgc tagcgctata        240

tgcgttgatg caatttctat gcgcacccgt tctcggagca ctgtccgacc gctttggccg 300

ccgcccagtc ctgctcgctt cgctacttgg agccactatc gactacgcga tcatggcgac 360

cacacccgtc ctgtggatcc tctacgccgg acgcatcgtg gccggcatca ccggcgccac 420

aggtgcggtt gctggcgcct atatcgccga catcaccgat ggggaagatc gggctcgcca 480

cttcgggctc atgagcgctt gtttcggcgt gggtatggtg gcaggccccg tggccggggg 540

actgttgggc gccatctcct tgcatgcacc attccttgcg gcggcggtgc tcaacggcct 600

caacctacta ctgggctgct tcctaatgca ggagtcgcat aagggagagc gtcgaccgat 660

gcccttgaga gccttcaacc cagtcagctc cttccggtgg gcgcggggca tgactatcgt 720

cgccgcactt atgactgtct tctttatcat gcaactcgta ggacaggtgc cggcagcgct 780

ctgggtcatt ttcggcgagg accgctttcg ctggagcgcg acgatgatcg gcctgtcgct 840

tgcggtattc ggaatcttgc acgccctcgc tcaagccttc gtcactggtc ccgccaccaa 900

acgtttcggc gagaagcagg ccattatcgc cggcatggcg gccgacgcgc tgggctacgt 960

cttgctggcg ttcgcgacgc gaggctggat ggccttcccc attatgattc ttctcgcttc 1020

cggcggcatc gggatgcccg cgttgcaggc catgctgtcc aggcaggtag atgacgacca 1080

tcagggacag cttcaaggat cgctcgcggc tcttaccagc ctaacttcga tcactggacc 1140

gctgatcgtc acggcgattt atgccgcctc ggcgagcaca tggaacgggt tggcatggat 1200

tgtaggcgcc gccctatacc ttgtctgcct ccccgcgttg cgtcgcggtg catggagccg 1260

ggccacctcg acctgaatgg aagccggcgg cacctcgcta acggattcac cactccaaga 1320

attggagcca atcaattctt gcggagaact gtgaatgcgc aaaccaaccc ttggcagaac 1380

atatccatcg cgtccgccat ctccagcagc cgcacgcggc gcatctcggg cagcgttggg 1440

tcctggccac gggtgcgcat gatcgtgctc ctgtcgttga ggacccggct aggctggcgg 1500

ggttgcctta ctggttagca gaatgaatca ccgatacgcg agcgaacgtg aagcgactgc 1560

tgctgcaaaa cgtctgcgac ctgagcaaca acatgaatgg tcttcggttt ccgtgtttcg 1620

taaagtctgg aaacgcggaa gtcagcgccc tgcaccatta tgttccggat ctgcatcgca 1680

ggatgctgct ggctaccctg tggaacacct acatctgtat taacgaagcg ctggcattga 1740

ccctgagtga tttttctctg gtcccgccgc atccataccg ccagttgttt accctcacaa 1800

cgttccagta accgggcatg ttcatcatca gtaacccgta tcgtgagcat cctctctcgt 1860

ttcatcggta tcattacccc catgaacaga aatccccctt acacggaggc atcagtgacc 1920

aaacaggaaa aaaccgccct taacatggcc cgctttatca gaagccagac attaacgctt 1980

ctggagaaac tcaacgagct ggacgcggat gaacaggcag acatctgtga atcgcttcac 2040

gaccacgctg atgagcttta ccgcagctgc ctcgcgcgtt tcggtgatga cggtgaaaac 2100

ctctgacaca tgcagctccc ggagacggtc acagcttgtc tgtaagcgga tgccgggagc 2160

agacaagccc gtcagggcgc gtcagcgggt gttggcgggt gtcggggcgc agccatgacc 2220

cagtcacgta gcgatagcgg agtgtatact ggcttaacta tgcggcatca gagcagattg 2280

tactgagagt gcaccatatg cggtgtgaaa taccgcacag atgcgtaagg agaaaatacc 2340

gcatcaggcg ctcttccgct tcctcgctca ctgactcgct gcgctcggtc gttcggctgc 2400

ggcgagcggt atcagctcac tcaaaggcgg taatacggtt atccacagaa tcaggggata 2460

acgcaggaaa gaacatgtga gcaaaaggcc agcaaaaggc caggaaccgt aaaaaggccg 2520

cgttgctggc gtttttccat aggctccgcc cccctgacga gcatcacaaa aatcgacgct 2580

caagtcagag gtggcgaaac ccgacaggac tataaagata ccaggcgttt ccccctggaa 2640

gctccctcgt gcgctctcct gttccgaccc tgccgcttac cggatacctg tccgcctttc 2700

tcccttcggg aagcgtggcg ctttctcata gctcacgctg taggtatctc agttcggtgt 2760

aggtcgttcg ctccaagctg ggctgtgtgc acgaaccccc cgttcagccc gaccgctgcg 2820

ccttatccgg taactatcgt cttgagtcca acccggtaag acacgactta tcgccactgg 2880

cagcagccac tggtaacagg attagcagag cgaggtatgt aggcggtgct acagagttct 2940

tgaagtggtg gcctaactac ggctacacta gaaggacagt atttggtatc tgcgctctgc 3000

tgaagccagt taccttcgga aaaagagttg gtagctcttg atccggcaaa caaaccaccg 3060

ctggtagcgg tggttttttt gtttgcaagc agcagattac gcgcagaaaa aaaggatctc 3120

aagaagatcc tttgatcttt tctacggggt ctgacgctca gtggaacgaa aactcacgtt 3180

aagggatttt ggtcatgaga ttatcaaaaa ggatcttcac ctagatcctt ttaaattaaa 3240

aatgaagttt taaatcaatc taaagtatat atgagtaaac ttggtctgac agttaccaat 3300

gcttaatcag tgaggcacct atctcagcga tctgtctatt tcgttcatcc atagttgcct 3360

gactccccgt cgtgtagata actacgatac gggagggctt accatctggc cccagtgctg 3420

caatgatacc gcgagaccca cgctcaccgg ctccagattt atcagcaata aaccagccag 3480

ccggaagggc cgagcgcaga agtggtcctg caactttatc cgcctccatc cagtctatta 3540

attgttgccg ggaagctaga gtaagtagtt cgccagttaa tagtttgcgc aacgttgttg 3600

ccattgctgc aggcatcgtg gtgtcacgct cgtcgtttgg tatggcttca ttcagctccg 3660

gttcccaacg atcaaggcga gttacatgat cccccatgtt gtgcaaaaaa gcggttagct 3720

ccttcggtcc tccgatcgtt gtcagaagta agttggccgc agtgttatca ctcatggtta 3780

tggcagcact gcataattct cttactgtca tgccatccgt aagatgcttt tctgtgactg 3840

gtgagtactc aaccaagtca ttctgagaat agtgtatgcg gcgaccgagt tgctcttgcc 3900

cggcgtcaac acgggataat accgcgccac atagcagaac tttaaaagtg ctcatcattg 3960

gaaaacgttc ttcggggcga aaactctcaa ggatcttacc gctgttgaga tccagttcga 4020

tgtaacccac tcgtgcaccc aactgatctt cagcatcttt tactttcacc agcgtttctg 4080

ggtgagcaaa aacaggaagg caaaatgccg caaaaaaggg aataagggcg acacggaaat 4140

gttgaatact catactcttc ctttttcaat attattgaag catttatcag ggttattgtc 4200

tcatgagcgg atacatattt gaatgtattt agaaaaataa acaaataggg gttccgcgca 4260

catttccccg aaaagtgcca cctgacgtct aagaaaccat tattatcatg acattaacct 4320

ataaaaatag gcgtatcacg aggccctttc gtcttcaaga a 4361

<210> 8
<211> 2501
<212> DNA
<213> Artificial

<220>
<223> Cloning vector pBR322

<400> 8
tcctctctcg tttcatcggt atcattaccc ccatgaacag aaatccccct tacacggagg 60
catcagtgac caaacaggaa aaaaccgccc ttaacatggc ccgctttatc agaagccaga 120
cattaacgct tctggagaaa ctcaacgagc tggacgcgga tgaacaggca gacatctgtg 180
aatcgcttca cgaccacgct gatgagcttt accgcagctg cctcgcgcgt ttcggtgatg 240
acggtgaaaa cctctgacac atgcagctcc cggagacggt cacagcttgt ctgtaagcgg 300
atgccgggag cagacaagcc cgtcagggcg cgtcagcggg tgttggcggg tgtcggggcg 360
cagccatgac ccagtcacgt agcgatagcg gagtgtatac tggcttaact atgcggcatc 420
agagcagatt gtactgagag tgcaccatat gcggtgtgaa ataccgcaca gatgcgtaag 480
gagaaaatac cgcatcaggc gctcttccgc ttcctcgctc actgactcgc tgcgctcggt 540
cgttcggctg cggcgagcgg tatcagctca ctcaaaggcg gtaatacggt tatccacaga 600
atcaggggat aacgcaggaa agaacatgtg agcaaaaggc cagcaaaagg ccaggaaccg 660
taaaaaggcc gcgttgctgg cgtttttcca taggctccgc ccccctgacg agcatcacaa 720
aaatcgacgc tcaagtcaga ggtggcgaaa cccgacagga ctataaagat accaggcgtt 780
tccccctgga agctccctcg tgcgctctcc tgttccgacc ctgccgctta ccggatacct 840
gtccgccttt ctcccttcgg gaagcgtggc gctttctcat agctcacgct gtaggtatct 900
cagttcggtg taggtcgttc gctccaagct gggctgtgtg cacgaacccc ccgttcagcc 960
cgaccgctgc gccttatccg gtaactatcg tcttgagtcc aacccggtaa gacacgactt 1020
atcgccactg gcagcagcca ctggtaacag gattagcaga gcgaggtatg taggcggtgc 1080

tacagagttc ttgaagtggt ggcctaacta cggctacact agaaggacag tatttggtat 1140

ctgcgctctg ctgaagccag ttaccttcgg aaaaagagtt ggtagctctt gatccggcaa 1200

acaaaccacc gctggtagcg gtggtttttt tgtttgcaag cagcagatta cgcgcagaaa 1260

aaaaggatct caagaagatc ctttgatctt ttctacgggg tctgacgctc agtggaacga 1320

aaactcacgt taagggattt tggtcatgag attatcaaaa aggatcttca cctagatcct 1380

tttaaattaa aaatgaagtt ttaaatcaat ctaaagtata tatgagtaaa cttggtctga 1440

cagttaccaa tgcttaatca gtgaggcacc tatctcagcg atctgtctat ttcgttcatc 1500

catagttgcc tgactccccg tcgtgtagat aactacgata cgggagggct taccatctgg 1560

ccccagtgct gcaatgatac cgcgagaccc acgctcaccg gctccagatt tatcagcaat 1620

aaaccagcca gccggaaggg ccgagcgcag aagtggtcct gcaactttat ccgcctccat 1680

ccagtctatt aattgttgcc gggaagctag agtaagtagt tcgccagtta atagtttgcg 1740

caacgttgtt gccattgctg caggcatcgt ggtgtcacgc tcgtcgtttg gtatggcttc 1800

attcagctcc ggttcccaac gatcaaggcg agttacatga tcccccatgt tgtgcaaaaa 1860

agcggttagc tccttcggtc ctccgatcgt tgtcagaagt aagttggccg cagtgttatc 1920

actcatggtt atggcagcac tgcataattc tcttactgtc atgccatccg taagatgctt 1980

ttctgtgact ggtgagtact caaccaagtc attctgagaa tagtgtatgc ggcgaccgag 2040

ttgctcttgc ccggcgtcaa cacgggataa taccgcgcca catagcagaa ctttaaaagt 2100

gctcatcatt ggaaaacgtt cttcggggcg aaaactctca aggatcttac cgctgttgag 2160

atccagttcg atgtaaccca ctcgtgcacc caactgatct tcagcatctt ttactttcac 2220

cagcgtttct gggtgagcaa aaacaggaag gcaaaatgcc gcaaaaaagg gaataagggc 2280

gacacggaaa tgttgaatac tcatactctt cctttttcaa tattattgaa gcatttatca 2340

gggttattgt ctcatgagcg gatacatatt tgaatgtatt tagaaaaata aacaaatagg 2400

ggttccgcgc acatttcccc gaaaagtgcc acctgacgtc taagaaacca ttattatcat 2460

gacattaacc tataaaaata ggcgtatcac gaggcccttt c 2501

<210> 9
<211> 5115
<212> DNA
<213> Artificial

<220>
<223> Cloning vector pQBI T7-GFP

<400> 9
ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc cggatatagt tcctcctttc agcaaaaaac ccctcaagac ccgtttagag 240

gccccaaggg gttatgctag ttattgctca gcggtggcag cagccaactc agcttccttt 300

cgggctttgt tagcagccgg atcctcagtt gtacagttca tccatgccat gtgtaatccc 360

agcagctgtt acaaactcaa gaaggaccat gtggtctctc ttttcgttgg gatctttcga 420

aagggcagat tgtgtggaca ggtaatggtt gtctggtaaa aggacagggc catcgccaat 480

tggagtattt tgttgataat ggtctgctag ttgaacgctt ccatcttcaa tgttgtggcg 540

ggtcttgaag ttcactttga ttccattctt ttgtttgtct gccatgatgt atacattgtg 600

tgagttatag ttgtattcca atttgtgtcc cagaatgttg ccatcttcct tgaagtcaat 660

accttttaac tcgattctat taacaagggt atcaccttca aacttgactt cagcacgtgt 720

cttgtagttg ccgtcatctt tgaagaagat ggtcctttcc tgtacataac cttcgggcat 780

ggcactcttg aaaaagtcat gccgtttcat atgatccggg tatcttgaaa agcattgaac 840

accatagcac agagtagtga ctagtgttgg ccatggaaca ggcagtttgc cagtagtgca 900

gatgaacttc agggtaagtt ttccgtatgt tgcatcacct tcaccctctc cactgacaga 960

gaacttgtgg ccgttaacat caccatctaa ttcaacaaga attgggacaa ctccagtgaa 1020

gagttcttct cctttgctag ccatatgtat atctccttct taaagttaaa caaaattatt 1080

tctagagggg aattgttatc cgctcacaat tcccctatag tgagtcgtat taatttcgcg 1140

ggatcgagat ctcgatcctc tacgccggac gcatcgtggc cggcatcacc ggcgccacag 1200

gtgcggttgc tggcgcctat atcgccgaca tcaccgatgg ggaagatcgg gctcgccact 1260

tcgggctcat gagcgcttgt ttcggcgtgg gtatggtggc aggccccgtg gccgggggac 1320

tgttgggcgc catctccttg catgcaccat tccttgcggc ggcggtgctc aacggcctca 1380

acctactact gggctgcttc ctaatgcagg agtcgcataa gggagagcgt cgagatcccg 1440

gacaccatcg aatggcgcaa aacctttcgc ggtatggcat gatagcgccc ggaagagagt 1500

caattcaggg tggtgaatgt gaaaccagta acgttatacg atgtcgcaga gtatgccggt 1560

gtctcttatc agaccgtttc ccgcgtggtg aaccaggcca gccacgtttc tgcgaaaacg 1620

cgggaaaaag tggaagcggc gatggcggag ctgaattaca ttcccaaccg cgtggcacaa 1680

caactggcgg gcaaacagtc gttgctgatt ggcgttgcca cctccagtct ggccctgcac 1740

gcgccgtcgc aaattgtcgc ggcgattaaa tctcgcgccg atcaactggg tgccagcgtg 1800

gtggtgtcga tggtagaacg aagcggcgtc gaagcctgta aagcggcggt gcacaatctt 1860

ctcgcgcaac gcgtcagtgg gctgatcatt aactatccgc tggatgacca ggatgccatt 1920

gctgtggaag ctgcctgcac taatgttccg gcgttatttc ttgatgtctc tgaccagaca 1980

cccatcaaca gtattatttt ctcccatgaa gacggtacgc gactgggcgt ggagcatctg 2040

gtcgcattgg gtcaccagca aatcgcgctg ttagcgggcc cattaagttc tgtctcggcg 2100

cgtctgcgtc tggctggctg gcataaatat ctcactcgca atcaaattca gccgatagcg 2160

gaacgggaag gcgactggag tgccatgtcc ggttttcaac aaaccatgca aatgctgaat 2220

gagggcatcg ttcccactgc gatgctggtt gccaacgatc agatggcgct gggcgcaatg 2280

cgcgccatta ccgagtccgg gctgcgcgtt ggtgcggata tctcggtagt gggatacgac 2340

gataccgaag acagctcatg ttatatcccg ccgttaacca ccatcaaaca ggattttcgc 2400

ctgctggggc aaaccagcgt ggaccgcttg ctgcaactct ctcagggcca ggcggtgaag 2460

ggcaatcagc tgttgcccgt ctcactggtg aaaagaaaaa ccaccctggc gcccaatacg 2520

caaaccgcct ctccccgcgc gttggccgat tcattaatgc agctggcacg acaggtttcc 2580

cgactggaaa gcgggcagtg agcgcaacgc aattaatgta agttagctca ctcattaggc 2640

accgggatct cgaccgatgc ccttgagagc cttcaaccca gtcagctcct tccggtgggc 2700

gcggggcatg actatcgtcg ccgcacttat gactgtcttc tttatcatgc aactcgtagg 2760

acaggtgccg gcagcgctct gggtcatttt cggcgaggac cgctttcgct ggagcgcgac 2820

gatgatcggc ctgtcgcttg cggtattcgg aatcttgcac gccctcgctc aagccttcgt 2880

cactggtccc gccaccaaac gtttcggcga gaagcaggcc attatcgccg gcatggcggc 2940

cgacgcgctg ggctacgtct tgctggcgtt cgcagtcacg tagcgatagc ggagtgtata 3000

ctggcttaac tatgcggcat cagagcagat tgtactgaga gtgcaccata tatgcggtgt 3060

gaaataccgc acagatgcgt aaggagaaaa taccgcatca ggcgctcttc cgcttcctcg 3120

ctcactgact cgctgcgctc ggtcgttcgg ctgcggcgag cggtatcagc tcactcaaag 3180

gcggtaatac ggttatccac agaatcaggg gataacgcag gaaagaacat gtgagcaaaa 3240

ggccagcaaa aggccaggaa ccgtaaaaag gccgcgttgc tggcgttttt ccataggctc 3300

cgcccccctg acgagcatca caaaaatcga cgctcaagtc agaggtggcg aaacccgaca 3360

ggactataaa gataccaggc gtttccccct ggaagctccc tcgtgcgctc tcctgttccg 3420

accctgccgc ttaccggata cctgtccgcc tttctccctt cgggaagcgt ggcgctttct 3480

catagctcac gctgtaggta tctcagttcg gtgtaggtcg ttcgctccaa gctgggctgt 3540

gtgcacgaac cccccgttca gcccgaccgc tgcgccttat ccggtaacta tcgtcttgag 3600

tccaacccgg taagacacga cttatcgcca ctggcagcag ccactggtaa caggattagc 3660

agagcgaggt atgtaggcgg tgctacagag ttcttgaagt ggtggcctaa ctacggctac 3720

actagaagga cagtatttgg tatctgcgct ctgctgaagc cagttacctt cggaaaaaga 3780

gttggtagct cttgatccgg caaacaaacc accgctggta gcggtggttt ttttgtttgc 3840

aagcagcaga ttacgcgcag aaaaaaagga tctcaagaag atcctttgat cttttctacg 3900

gggtctgacg ctcagtggaa cgaaaactca cgttaaggga ttttggtcat gagattatca 3960

aaaaggatct tcacctagat ccttttaaat taaaaatgaa gttttaaatc aatctaaagt 4020

atatatgagt aaacttggtc tgacagttac caatgcttaa tcagtgaggc acctatctca 4080

gcgatctgtc tatttcgttc atccatagtt gcctgactcc ccgtcgtgta gataactacg 4140

atacgggagg gcttaccatc tggccccagt gctgcaatga taccgcgaga cccacgctca 4200

ccggctccag atttatcagc aataaaccag ccagccggaa gggccgagcg cagaagtggt 4260

cctgcaactt tatccgcctc catccagtct attaattgtt gccgggaagc tagagtaagt 4320

agttcgccag ttaatagttt gcgcaacgtt gttgccattg ctgcaggcat cgtggtgtca 4380

cgctcgtcgt ttggtatggc ttcattcagc tccggttccc aacgatcaag gcgagttaca 4440

tgatccccca tgttgtgcaa aaaagcggtt agctccttcg gtcctccgat cgttgtcaga 4500

agtaagttgg ccgcagtgtt atcactcatg gttatggcag cactgcataa ttctcttact 4560

gtcatgccat ccgtaagatg cttttctgtg actggtgagt actcaaccaa gtcattctga 4620

gaatagtgta tgcggcgacc gagttgctct tgcccggcgt caacacggga taataccgcg 4680

ccacatagca gaactttaaa agtgctcatc attggaaaac gttcttcggg gcgaaaactc 4740

tcaaggatct taccgctgtt gagatccagt tcgatgtaac ccactcgtgc acccaactga 4800

tcttcagcat cttttacttt caccagcgtt tctgggtgag caaaaacagg aaggcaaaat 4860

gccgcaaaaa agggaataag ggcgacacgg aaatgttgaa tactcatact cttccttttt 4920

caatattatt gaagcattta tcagggttat tgtctcatga gcggatacat atttgaatgt 4980

atttagaaaa ataaacaaat aggggttccg cgcacatttc cccgaaaagt gccacctgac 5040

gtctaagaaa ccattattat catgacatta acctataaaa ataggcgtat cacgaggccc 5100

tttcgtcttc aagaa 5115

<210> 10
<211> 1031
<212> DNA
<213> Artificial

<220>
<223> Cloning vector pQBI T7-GFP

<400> 10
ctggatgctg taggcatagg cttggttatg ccggtactgc cgggcctctt gcgggatatc 60

cggatatagt tcctcctttc agcaaaaaac ccctcaagac ccgtttagag gccccaaggg 120

gttatgctag ttattgctca gcggtggcag cagccaactc agcttccttt cgggctttgt 180

tagcagccgg atcctcagtt gtacagttca tccatgccat gtgtaatccc agcagctgtt 240

acaaactcaa gaaggaccat gtggtctctc ttttcgttgg gatctttcga aagggcagat 300

tgtgtggaca ggtaatggtt gtctggtaaa aggacagggc catcgccaat tggagtattt 360

tgttgataat ggtctgctag ttgaacgctt ccatcttcaa tgttgtggcg ggtcttgaag 420

ttcactttga ttccattctt ttgtttgtct gccatgatgt atacattgtg tgagttatag 480

ttgtattcca atttgtgtcc cagaatgttg ccatcttcct tgaagtcaat accttttaac 540

tcgattctat taacaagggt atcaccttca aacttgactt cagcacgtgt cttgtagttg 600

ccgtcatctt tgaagaagat ggtcctttcc tgtacataac cttcgggcat ggcactcttg 660

aaaaagtcat gccgtttcat atgatccggg tatcttgaaa agcattgaac accatagcac 720

agagtagtga ctagtgttgg ccatggaaca ggcagtttgc cagtagtgca gatgaacttc 780

agggtaagtt ttccgtatgt tgcatcacct tcaccctctc cactgacaga gaacttgtgg 840

ccgttaacat caccatctaa ttcaacaaga attgggacaa ctccagtgaa gagttcttct 900

cctttgctag ccatatgtat atctccttct taaagttaaa caaaattatt tctagagggg 960

aattgttatc cgctcacaat tcccctatag tgagtcgtat taatttcgcg ggatcgagat 1020

ctcgatcctc t 1031

<210> 11
<211> 33
<212> DNA
<213> Artificial

<220>
<223> Synthetic DNA

<400> 11
accgccacct ccgttgtaca gttcatccat gcc 33

<210> 12
<211> 34
<212> DNA
<213> Artificial

<220>
<223> Synthetic DNA

<400>  12
aggatccggc tgctaacaaa gcccgaaagg aagc                                    34

<210>  13
<211>  34
<212>  DNA
<213>  Artificial

<220>
<223>  Synthetic DNA

<400>  13
agccggatcc tcagttgtac agttcatcca tgcc                                    34

<210>  14
<211>  36
<212>  DNA
<213>  Artificial

<220>
<223>  Synthetic DNA

<400>  14
aggtggcggt ggcaaaggag aagaactctt cactgg                                  36

<210>  15
<211>  701
<212>  DNA
<213>  Artificial

<220>
<223>  Cloning vector pQBI 67 (BFP gene)

<400> 15
agttcatcca tgccatgtgt aatcccagca gctgttacaa actcaagaag gaccatgtgg 60
tctctctttt cgttgggatc tttcgaaagg gcagattgtg tggacaggta atggttgtct 120
ggtaaaagga cagggccatc gccaattgga gtattttgtt gataatggtc tgctagttga 180
acgcttccat cttcaatgtt gtggcggatc ttgaagttcg ctttgattcc attcttttgt 240
ttgtctgcca tgatgtatac attgtgtgag ttatagttgt attccaattt gtgtccaaga 300
atgtttccat cttctttaaa atcaatacct tttaactcga ttctattaac aagggtatca 360
ccttcaaact tgacttcagc acgtgtcttg tagttgccgt catctttgaa gaagatggtc 420
ctttcctgta cataaccttc gggcatggca ctcttgaaaa agtcatgccg tttcatatga 480
tccgggtatc ttgaaaagca ttgaacacca tgagagagag tagtgactag tgttggccat 540
ggaacaggca gtttgccagt agtgcagatg aacttcaggg taagttttcc gtatgttgca 600
tcaccttcac cctctccact gacagagaac ttgtggccgt taacatcacc atctaattca 660
acaagaattg ggacaactcc agtgaagagt tcttctcctt t 701

## Description

### TECHNICAL FIELD

The present invention relates to a method for directly preparing a desired double-stranded DNA fragment having a sticky end (a protruding end) without a restriction enzyme digestion or the like. Moreover, the present invention also relates to a genetic recombination method using the DNA fragment obtained by the aforementioned method.

### BACKGROUND ART

Conventionally, in the field of molecular biology and biotechnology, a plasmid DNA is cleaved by restriction enzymes, and the cleavage portion used as a host DNA is allowed to bind to a desired gene fragment using a ligase, so as to construct a vector. In many cases, the aforementioned gene fragment is prepared from various materials (template DNAs) by PCR, and in order to bind it to a plasmid DNA (a host DNA), the blunt ends of the PCR amplification product are digested with restriction enzymes, so that they are converted to sticky ends (protruding ends) (Sambrook, J. et al., 2001, Molecular Cloning: A Laboratory Manual Edn.3. Cold Spring Harbor Laboratory Press, Cold Spring Harbor, NY). By this technique, a desired vector can be obtained at a relatively high efficiency. However, this technique has the following two problems. The first problem is that, since the restriction site is limited to several palindromes, it may be often difficult to find out restriction enzymes suitable for cleaving DNA at a site of interest (or around such site of interest). The second problem is that a majority of available restriction enzymes recognize a DNA sequence consisting of 4 to 6 bases, and that, when a DNA with a large size is digested with restriction enzymes (wherein many identical recognition sites exist), many sites other than a desired site are also cleaved. As a result, if a host DNA is large in size, precise genetic recombination operations cannot be conducted.

As a solution to such problems, the present inventor has combined a Ce4⁺/EDTA complex (molecular scissors) with a pair of pseudo-complementary peptide nucleic acids (pcPNAs) (invasion in a double-stranded DNA and production of a locally single-stranded region), so as to produce an artificial restriction DNA cutter (ARCUT). If such pcPNAs are allowed to act on a double-stranded DNA, the pcPNAs partially dissociate the double-stranded DNA and then bind it to their target sequence (inversion in a double-stranded DNA). Herein, if the target sequence of the pcPNAs is shifted by several bases, a side opposite to the pcPNA-binding portion locally remains as a single strand during the formation of a complex. If Ce⁴⁺/EDTA is allowed to act on this complex, the Ce⁴⁺/EDTA cleaves a single-stranded DNA more selectively than a double-stranded DNA. Thus, the single-stranded portion produced by the pcPNAs is selectively cleaved. Since the cleavage site of each DNA strand is shifted in the horizontal direction, a sticky end is formed after completion of the cleavage. The sticky end formed by ARCUT does not directly bind to the ends of other fragments that have been separately prepared (e.g. a restriction fragment). However, with the use of a joint oligonucleotide that fills the space between these fragments, the fragments apparently form a complementary structure, and they may bind to each other (Yamamoto, Y. et al., Chem. Bio. Chem., vol.7, pp. 673-677, 2006). However, the method using ARCUT has been problematic in that it has low binding efficiency (ligation efficiency) and in that it becomes difficult to carry out purification/separation steps.

### DISCLOSURE OF THE INVENTION

Thus, it is an object of the present invention to provide a method for preparing a DNA fragment, in which a desired double-stranded DNA fragment having a sticky end is directly and easily obtained from an amplification product (an amplified fragment) after PCR without a restriction enzyme digestion. Moreover, it is another object of the present invention to provide a genetic recombination method and the like using the DNA fragment obtained by the above-described preparation method.

The present inventor has conducted intensive studies directed towards achieving the aforementioned objects. As a result, the inventor has found that the aforementioned objects can be achieved with the use of a primer used for PCR, which has a protecting group having a specific function, thereby completing the present invention.

Specifically, the present invention is as follows:
(1) A primer used for PCR, which is composed of a complementary DNA portion consisting of a nucleotide sequence complementarily binding to an amplification target region in a template DNA and a non-complementary DNA portion consisting of a nucleotide sequence that links to the 5' end of the complementary DNA portion but does not complementarily bind to the amplification target sequence, wherein at least a base corresponding to the 3' end in the nucleotide sequence of the non-complementary DNA portion is modified with a protecting group capable of terminating the progression of DNA replication catalyzed by a DNA polymerase.
   In the primer of the present invention, the protecting group can be dissociated from the modified base, for example, by a light irradiation treatment, an alkali treatment, an acid treatment, an oxidation treatment, a reduction treatment, a desilylation treatment, a heat treatment, an esterase treatment or a phosphatase treatment.
   Herein, examples of the protecting group that can be dissociated from the modified base by the light irradiation treatment include a 2-(2-nitrophenyl)propyl group, a 2-(2-nitrophenyl)propyloxymethyl group, a 1-(2-nitrophenyl)ethyl group and a 6-nitropiperonyloxymethyl group. Examples of the protecting group that can be dissociated from the modified base by the alkali treatment include an isobutyryl group, a benzoyl group and an acetoxymethyl group. Examples of the protecting group that can be dissociated from the modified base by the acid treatment include a trityl group and a methoxy derivative thereof. Examples of the protecting group that can be dissociated from the modified base by the oxidation treatment include an allyloxymethyl group, a dimethoxybenzyloxymethyl group and a trimethoxybenzyloxymethyl group. Examples of the protecting group that can be dissociated from the modified base by the reduction treatment include a benzyloxymethyl group, and a benzyloxymethyl group substituted with any given substituent. Examples of the protecting group that can be dissociated from the modified base by the desilylation treatment include a t-butyldimethoxysilyloxymethyl group and a t-butyldiphenylsilyloxymethyl group. An example of the protecting group that can be dissociated from the modified base by the heat treatment is an isocyanate group. An example of the protecting group that can be dissociated from the modified base by the esterase treatment is an acetoxymethyl group. An example of the protecting group that can be dissociated from the modified base by the phosphatase treatment is a methyl phosphate group.
   Moreover, in the primer of the present invention, examples of at least a base corresponding to the 3' end in the nucleotide sequence of the non-complementary DNA portion include thymine and guanine. Furthermore, in the primer of the present invention, the non-complementary DNA portion is, for example, 1 to 100 bases in length.
(2) A method for preparing a DNA fragment having a sticky end, which comprises: (i) a step of performing a PCR reaction using a template DNA and the primer according to any one of claims 1 to 13 to obtain an amplified DNA fragment; and (ii) a step of performing a prescribed treatment on the amplified DNA fragment to dissociate a protecting group from the fragment.
   In the preparation method of the present invention, the prescribed treatment for dissociating the protecting group from the modified base is, for example, a light irradiation treatment, an alkali treatment, an acid treatment, an oxidation treatment, a reduction treatment, a desilylation treatment, a heat treatment, an esterase treatment or a phosphatase treatment.
   In the preparation method of the present invention, the sticky end is, for example, a sticky end having the same nucleotide sequence as that of a sticky end obtained by a restriction enzyme digestion, or a sticky end having a nucleotide sequence different from that of a sticky end obtained by a restriction enzyme digestion.
(3) A genetic recombination method, which comprises a step of binding the DNA fragment obtained by the preparation method according to (2) above to a host DNA.
   In the genetic recombination method of the present invention, the binding operation of the above-described DNA fragment to the host DNA can be carried out, for example, without using a DNA ligase.
(4) A substituent-introducing agent comprising a compound represented by the following formula (I'): 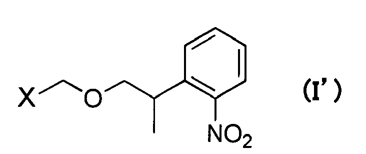 [wherein, in the formula (I'), X represents Cl, I, Br, p-toluenesulfonic acid ester or sulfuric acid ester].
(5) A biomolecule having a 2-(2-nitrophenyl)propyloxymethyl group.

An example of the biomolecule of the present invention is a biomolecule represented by the following formula (I): 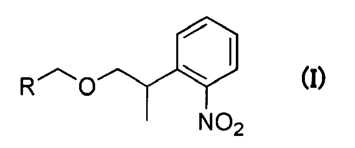 [wherein, in the formula (I), R represents a biomolecule].

Herein, an example of the biomolecule is a base, and specific examples include thymine and guanine. When the biomolecules are thymine and guanine, examples of the biomolecule of the present invention include those represented by the following formulae (II) and (III):

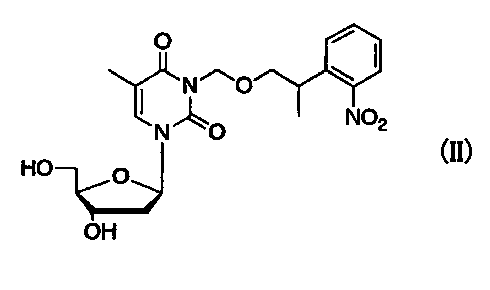

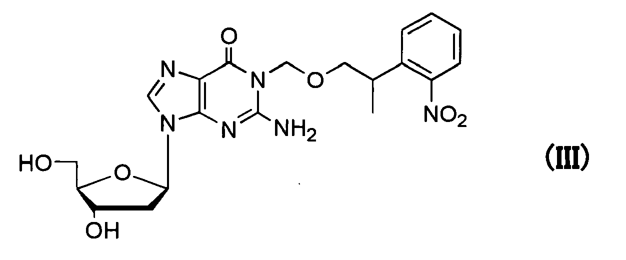

### BRIEF DESCRIPTION OF THE DRAWINGS

Figure 1 is a schematic view of LACE-PCR (Light-assisted-cohesive-ending PCR) using caged T^{NPP}, which is one example of the present invention.
Figure 2 is a photograph of an electrophoresis (20% denaturing PAGE) showing the results of an elongation reaction catalyzed by a DNA polymerase. Lane 1 shows only C2 (control), lane 2 shows C1 + C2 + Ex Taq (without UV irradiation), and lane 3 shows C1 + C2 + Ex Taq (with UV irradiation). The reaction conditions are [C1] = [C2] = 4 µM, [dNTPs] = 100 µM, [Ex Taq] = 0.2 U/µl, 37°C, and 30 minutes.
Figure 3 is a schematic view of the construction of a recombinant vector by the LACE-PCR method.
Figure 4 is a view showing the structure of an insert DNA fragment prepared by the LACE-PCR method.
Figure 5 is a photograph showing an electrophoresis of an amplification product by the LACE-PCR method.
Figure 6 is a photograph of an electrophoresis showing the results of colony PCR.
Figure 7 is a view showing the results of the analyses of the nucleotide sequences of recombinant vectors.
Figure 8 is a graph showing a change over time in the deprotection of a protecting group (NPP) by UV irradiation.
Figure 9 is a view showing the structure of an insert DNA fragment prepared by the LACE-PCR method.
Figure 10 is a photograph showing an electrophoresis of an amplification product by the LACE-PCR method, which is carried out before and after UV irradiation. Lane 1 shows before UV irradiation, and lane 2 shows after UV irradiation.
Figure 11 is a photograph of an electrophoresis showing the results of colony PCR.
Figure 12 is a view showing the results of the analyses of the nucleotide sequences of recombinant vectors. Figure 12(a) shows a recombinant vector, to which an *Eco* RI site has bound. Figure 12(b) shows a recombinant vector, to which a *Hind* III site has bound.
Figure 13 is a view showing target cleavage sites to be cleaved by ARCUT and the nucleotide sequences of pcPNAs. Figure 13(a) shows a nucleotide sequence around a target site to be cleaved by ARCUT. Figure 13(b) shows a structure around the target site that is cleaved by ARCUT.
Figure 14(a) is a view showing the structure of a target fragment (vector) cleaved by ARCUT, and Figure 14(b) is a view showing the structure of an insert DNA fragment (insert) prepared by the LACE-PCR method.
Figure 15 is a view showing a restriction site on a pBR322 vector.
Figure 16(a) is a view showing the structure of a target fragment (vector) cleaved by ARCUT, and Figure 16(b) is a view showing the structure of an insert DNA fragment (insert) prepared by the LACE-PCR method.
Figure 17 is a photograph showing an agarose gel electrophoresis of the cleavage fragment of a pBR322 vector cleaved by *Eco*RI or ARCUT. Lane 1 shows a fragment cleaved by only *Eco*RI, lane 2 shows a fragment cleaved by *Eco*RI and then by ARCUT, and lane M shows a 1 kbp ladder marker.
Figure 18 is a photograph showing an agarose gel electrophoresis of individual fragments used or obtained in the present Examples. Lane 1 shows an ARCUT cleavage fragment (2530 bp), lane 2 shows an amplification product obtained by the LACE-PCR method (with UV irradiation), lane 3 shows only a recombinant vector, and lane 4 shows a recombinant vector cleaved by *EcoRI.*
Figure 19 is a view showing the results of the analyses of the nucleotide sequences of recombinant vectors. Figure 19(a) shows a recombinant vector, to which an ARCUT site has bound. Figure 19(b) shows a recombinant vector, to which an *Eco* RI site has bound.
Figure 20 is a photograph showing GFP luminescence in a transformant of BL21-Gold (DE3).
Figure 21 is a schematic view showing the construction of a vector comprising a DNA encoding a GFP-BFP fusion protein. The left view shows a pQBI T7-GFP vector comprising a GFP gene, and the right view shows a pQBI 67 vector comprising a BFP gene.
Figure 22 is a photograph showing an agarose gel electrophoresis of a product obtained by amplifying a DNA fragment comprising a BFP gene by LACE-PCR.
Figure 23 is a photograph showing the luminescence of a GFP-BFP fusion protein or the like in a transformant of BL21-Gold (DE3).
Figure 24 is a photograph showing an agarose gel electrophoresis of a recombinant vector recovered from a transformant of BL21-Gold (DE3).
Figure 25 is a view showing the fluorescence spectrum of a GFP-BFP fusion protein.
Figure 26 is a photograph showing the results of SDS-PAGE analysis of a protein expressing in a transformant ofBL21-Gold (DE3).

### BEST MODE FOR CARRYING OUT THE INVENTION

The present invention will be described in detail below. The following descriptions are not intended to limit the scope of the present invention. Other than the following examples, the present invention may be modified and may be carried out, as appropriate, within a range that does not impair the intention of the present invention. The present specification includes all of the contents as disclosed in the specification of Japanese Patent Application No. 2008-061678 (filed on March 11, 2008), which is a priority document of the present application. Moreover, all publications cited in the present specification, which include prior art documents and patent documents such as laid-open application publications and patent publications, are incorporated herein by reference in their entirety.

### 1. Summary of the present invention

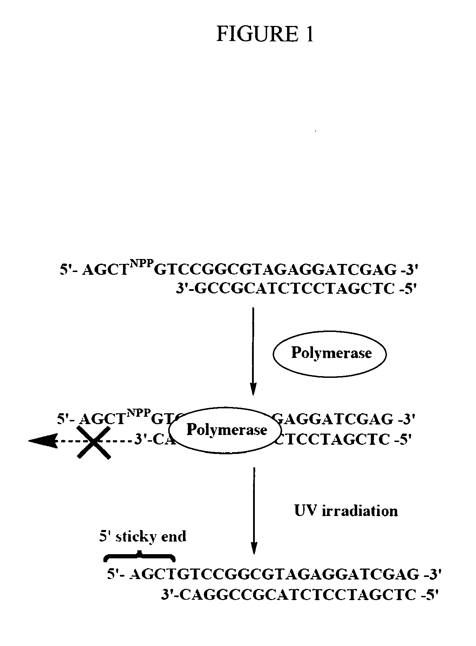

As shown in Figure 1, if a modification has been made to inhibit base-pair formation between dNTPs and a template DNA (T^{NPP}; a thymidine residue modified with (protected by) a 2-(2-nitrophenyl)propyl group), an elongation reaction catalyzed by a DNA polymerase is inhibited, and as a result, a DNA fragment having a 5' protruding end can be easily produced. The thus modified (protected) DNA fragment cannot be directly applied to genetic recombination. However, since a 2-(2-nitrophenyl)propyl group (NPP group) serving as a protecting group for T^{NPP} can be dissociated by light irradiation, the obtained DNA fragment can be easily used for genetic recombination technology and the like. The present inventor has found that this technique is applied in combination with a PCR technology, so as to create a method for arbitrarily designing a 5' protruding end (sticky end) on an insert side that can be used in genetic recombination and the like, thereby completing the present invention.

Hence, the present inventor has performed the aforementioned modification on some bases to synthesize various PCR primers (see a C1 primer shown in Scheme 1 of Example 1 as described later, for example). Thereafter, the inventor has inserted the obtained PCR amplification product acting as an insert fragment into a vector without a restriction enzyme digestion, so as to construct a recombinant vector. By the way, it has been known that, in the case of a DNA cleavage fragment cleaved by ARCUT (an artificial restriction DNA cutter; see Yamamoto, Y. et al., Chem. Bio. Chem., vol.7, pp. 673-677, 2006), due to the properties thereof, the nucleotide sequence of the cleavage portion must not be a sequence as in the case of using natural restriction enzymes. Thus, a vector is cleaved by ARCUT, and a DNA fragment serving as an insert is then prepared by the method of the present invention, such that it is matched with the nucleotide sequence of the aforementioned cleavage portion. Thereafter, the DNA fragment was attempted to be inserted into the vector. Conventionally, in genetic recombination using ARCUT, when an insert fragment has been ligated to a vector, it has been necessary to use an additional fragment called "Oligojoint" to fill the space between them. However, if the insert fragment prepared by the method of the present invention is used, such insert fragment could be extremely easily inserted into a vector without using Oligojoint. Several methods for producing a DNA fragment having a desired protruding end (sticky end) have been reported, so far. However, all of such methods have required a special enzyme digestion after completion of PCR (Aslanidis, C. et al., (1990) Ligation-independent cloning of PCR products (LIC-PCR). Nucleic Acids Res., 18, 6069-6074.; Bitinaite, J. et al., (2007) USERTM friendly DNA engineering and cloning method by uracil excision. Nucleic Acids Res., 35, 1992-2002.; Xin, W. et al., (2003) Construction of linear functional expression elements with DNA and RNA hybrid primers: a flexible method for proteomics., 25, 273-277.; Zhu, B. et al., (2007) In-Fusion™ assembly: seamless engineering of multidomain fusion proteins, modular vectors, and mutations. BioTechniques, 43, 354-359). According to the method of the present invention, a DNA fragment having a desired protruding end can be produced using substantially only a DNA polymerase as an enzyme. Since this method has a few limits (a few conditions), it is considered to be used for various applications.

### 2. Method for preparing DNA fragment having sticky end

The method for preparing a DNA fragment having a sticky end according to the present invention comprises the following steps (i) and (ii):
(i) a step of performing a PCR reaction using a template DNA and a prescribed primer used for PCR to obtain an amplified DNA fragment; and
(ii) a step of performing a prescribed treatment on the amplified DNA fragment to dissociate a protecting group from the fragment.

Herein, the prescribed PCR primer used in the aforementioned step (i) is a primer, which is composed of a complementary DNA portion consisting of a nucleotide sequence complementarily binding to an amplification target region in a template DNA that is a part of the template DNA, and a non-complementary DNA portion consisting of a nucleotide sequence that links to the 5' end of the complementary DNA portion but does not complementarily bind to the amplification target sequence; the primer being **characterized in that** at least a base corresponding to the 3' end in the nucleotide sequence of the non-complementary DNA portion is modified with a protecting group capable of terminating the progression of DNA replication catalyzed by a DNA polymerase. Herein, the "non-complementary DNA portion" in the concerned primer may be a portion consisting of a nucleotide sequence that is not complementary to the PCR amplification target region in the template DNA. Accordingly, the "non-complementary DNA portion" may also be a portion consisting of a nucleotide sequence complementary to a region other than the amplification target region in the template DNA (for example, a nucleotide sequence region with any given length adjacent to the 5' or 3' side of the amplification target region). Thus, the non-complementary DNA portion is not limited.

In the concerned primer, the non-complementary DNA portion is a portion that serves as a sticky end of the finally obtained DNA fragment (a single-stranded DNA portion). Herein, the base length of the non-complementary DNA portion is not particularly limited. It is, for example, 1 to 100 bases, and preferably 4 to 20 bases. In particular, according to the preparation method of the present invention, the base length of the sticky end of the obtained DNA fragment can be easily set at 5 bases or more, which cannot be generally realized by the sticky end after a restriction enzyme digestion. Accordingly, the sticky end of the obtained DNA fragment may consist of a nucleotide sequence that is either identical to or different from that of a sticky end obtained by a known restriction enzyme digestion. Thus, the sticky end is not limited.

The concerned primer has a protecting group capable of terminating the progression of DNA replication catalyzed by a DNA polymerase, which modifies the aforementioned prescribed base (at least a base corresponding to the 3' end in the nucleotide sequence of the non-complementary DNA portion). The type of the base is not limited. Thymine and guanine are preferable. In general, in PCR operations, a complementary strand is synthesized also for the non-complementary DNA portion in the primer during the process of DNA synthesis catalyzed by a DNA polymerase, and DNA replication progresses. In the present invention, however, since the base located between the complementary DNA portion and the non-complementary DNA portion in the primer is modified with the aforementioned protecting group, such complementary strand is not synthesized for the non-complementary DNA portion during the DNA synthesis process in PCR. Accordingly, the obtained DNA fragment has a sticky end that corresponds to the non-complementary DNA portion.

Herein, the aforementioned protecting group is not limited, as long as it can be dissociated from the DNA fragment obtained after PCR by the prescribed treatment. Preferred examples include protecting groups described in the cited document, Green et al. (Protective Groups in Organic Synthesis, John Wiley and Sons, 2nd edition, 1991). In the present invention, protecting groups that can be dissociated from the modified base by a light irradiation treatment, an alkali treatment, an acid treatment, an oxidation treatment, a reduction treatment, a desilylation treatment, a heat treatment, an esterase treatment or a phosphatase treatment, are preferable. Among these treatments, a light irradiation treatment (particularly preferably, a UV irradiation treatment), an alkali treatment, an oxidation treatment, a reduction treatment, a desilylation treatment and the like are more preferable. Accordingly, various types of treatments as listed above can be exemplified as prescribed treatments that are carried out in the above step (ii).

In the case of the above-described light irradiation treatment (particularly preferably, a UV irradiation treatment), for example, light is applied preferably for 5 to 60 minutes, and more preferably for 15 to 30 minutes, although the conditions depend on the type of a protecting group treated.

Hereinafter, protecting groups that can be dissociated by such light irradiation treatment will be exemplified. The groups enclosed with broken lines correspond to the concerned protecting groups.

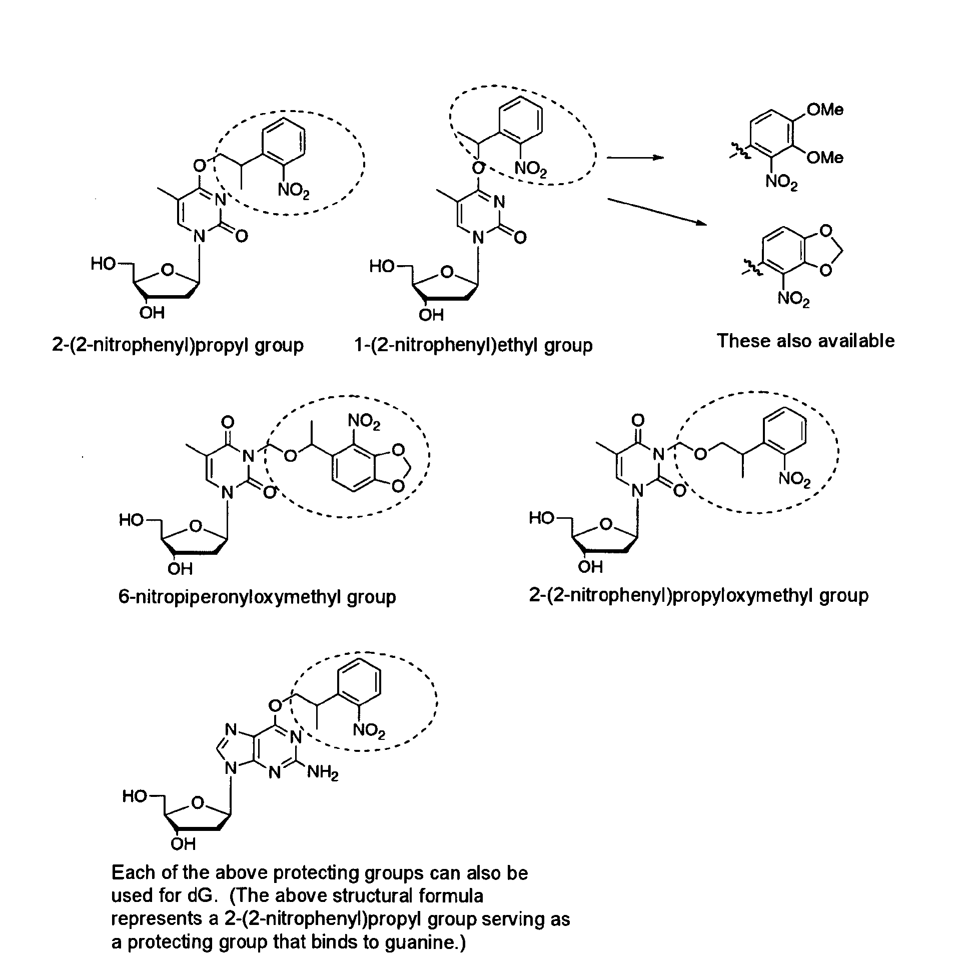

In the case of the above-described alkali treatment, concentrated ammonia water, a mixed solution of concentrated ammonia water and methylamine and the like are preferably used as reagents, for example. Concentrated ammonia water is more preferable. The alkali treatment is carried out, preferably at room temperature for 24 hours or at 55°C for 8 hours, and more preferably at room temperature for 24 hours, although the conditions depend on the type of a protecting group treated.

Hereinafter, protecting groups that can be dissociated by such alkali treatment will be exemplified. The groups enclosed with broken lines correspond to the concerned protecting groups.

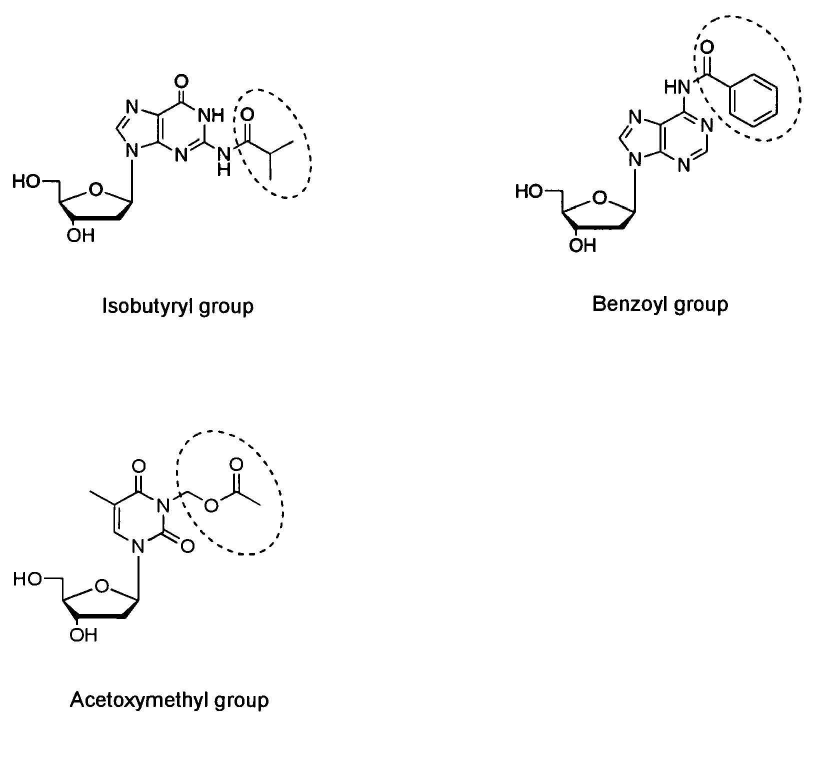

In the case of the above-described acid treatment, a 2%-50% trifluoroacetic acid aqueous solution, 10%-80% acetic acid, diluted hydrochloric acid and the like are preferably used as reagents, for example. A 2% trifluoroacetic acid aqueous solution and 80% acetic acid are more preferable. The acid treatment is carried out, preferably at room temperature for 10 minutes to overnight, and more preferably at room temperature for 1 hour, although the conditions depend on the type of a protecting group treated.

Preferred examples of a protecting group that can be dissociated by such acid treatment include a trityl group and a methoxy derivative thereof.

In the case of the above-described oxidation treatment, 2,3-dichloro-5,6-dicyano-p-benzoquinone, iodine and the like are preferably used as reagents, for example. 2,3-Dichloro-5,6-dicyano-p-benzoquinone is more preferable. The oxidation treatment is carried out, for example, by adding 2,3-dichloro-5,6-dicyano-p-benzoquinone to the sample in an aqueous solution, and then stirring it at room temperature, preferably for one hour to several days, and more preferably for 10 hours, although the conditions depend on the type of a protecting group treated.

Preferred examples of a protecting group that can be dissociated by such oxidation treatment include an allyloxymethyl group, a dimethoxybenzyloxymethyl group and a trimethoxybenzyloxymethyl group.

In the case of the above-described reduction treatment, with regard to reagents used, palladium-carbon, a Raney catalyst, an Adam's catalyst, a Lindlar's catalyst and the like are preferably used as catalysts combined with hydrogen gas, for example. Of these, palladium-carbon is more preferable. In addition, a single use of sodium borohydride or sodium cyanoborohydride is also preferable. The reduction treatment is carried out by adding a catalyst and hydrogen gas to a reaction system, and then reacting them at room temperature, preferably for overnight to several days, and more preferably overnight, although the conditions depend on the type of a protecting group treated.

Preferred examples of a protecting group that can be dissociated by such reduction treatment include a benzyloxymethyl group, and a benzyloxymethyl group which is substituted with any given substituent. Examples of such any given substituent include various types of known substituents such as those included in an alkyl group, an alkenyl group, an aryl group, an aralkyl group, a cycloalkyl group, an acyl group and a heterocyclic group. In the case of the above-described desilylation treatment, a 1 M tetrabutylammonium fluoride (THF) solution, triethylamine trihydrofluoride and the like are preferably used as reagents, for example. A 1 M tetrabutylammonium fluoride (THF) solution is more preferable. The desilylation treatment is carried out by adding a 1 M tetrabutylammonium fluoride (THF) solution to a sample solution, and then reacting them at room temperature, preferably for overnight to several days, and more preferably overnight.

Protecting groups that can be dissociated by such desilylation treatment will be exemplified. The groups enclosed with broken lines correspond to the concerned protecting groups.

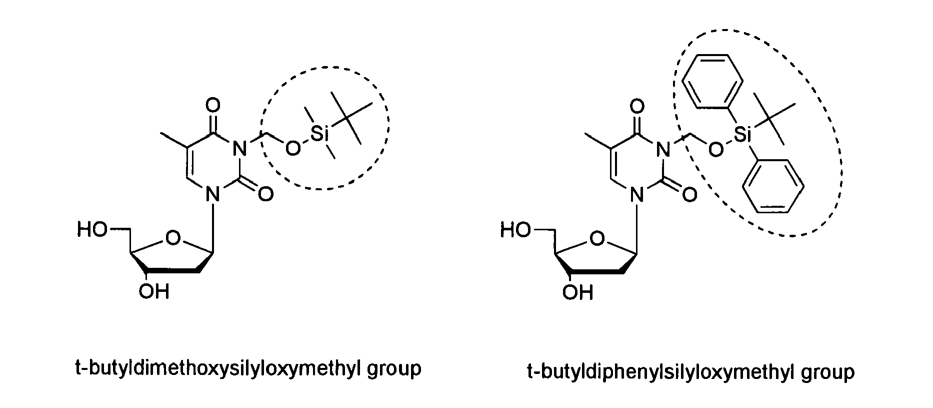

The above-described heat treatment is carried out, for example, by heating the reaction product comprising a protecting group, preferably at a temperature of 60°C to 90°C for 10 to 360 minutes, and more preferably at 60°C for 10 minutes, although the conditions depend on the type of the protecting group treated.

A preferred example of protecting groups that can be dissociated by such heat treatment is an isocyanate group (see JP Patent Publication (Kokai) No. 2006-248931 A).

The above-described esterase treatment is carried out, for example, by reacting the reaction product comprising a protecting group with pig liver esterase, preferably at 37°C for 1 hour or overnight, and more preferably at 37°C for 1 hour, although the conditions depend on the type of the protecting group treated.

A protecting group that can be dissociated by such esterase treatment will be exemplified below. The group enclosed with broken line corresponds to the concerned protecting group.

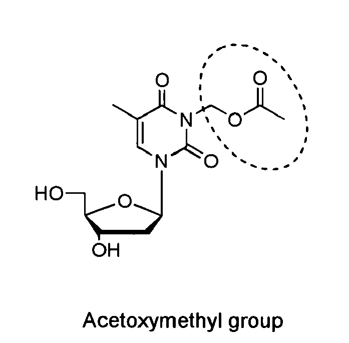

The above-described phosphatase treatment is carried out, for example, by reacting the reaction product comprising a protecting group with alkaline phosphatase, preferably at 37°C for 1 hour or overnight, and more preferably at 37°C for 1 hour, although the conditions depend on the type of the protecting group treated.

A protecting group that can be dissociated by such phosphatase treatment will be exemplified below. The group enclosed with broken line corresponds to the concerned protecting group.

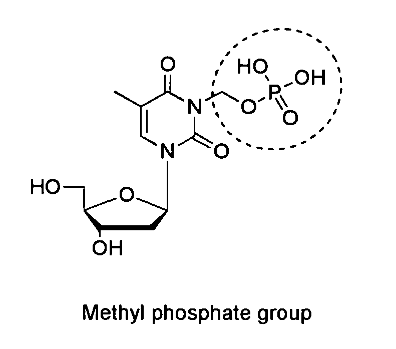

In the present invention, a known method for preparing a primer used for PCR having the aforementioned various types of protecting groups may be applied, and the preparation method is not limited. For example, the protection of a desired base by the aforementioned various types of protecting groups can be carried out: by activating with a tosyl compound, thymidine whose 5' and 3' hydroxyl groups have been protected by acetyl groups, and then allowing the alcohol form of a protecting group to act thereon; or by allowing a protecting group activated as a chloromethyl form to act on thymidine whose 5' and 3' hydroxyl groups have been protected by acetyl groups.

In the step (i) above, PCR conditions other than the above-described conditions, such as a reaction solution (a template concentration, a primer concentration, the type and concentration of a buffer, the type of a DNA polymerase, the amount of the enzyme, etc.) and reaction conditions (the reaction time and temperature of each of heat denaturation, annealing and elongation, the total cycle number, etc.), can be determined, as appropriate, based on known findings and common technical knowledge.

In the step (ii) above as well, the prescribed treatment for deprotection can be carried out by a known method, under known conditions, as well as the above-described conditions.

The preparation method of the present invention may also comprise other any given steps, as well as the above-described steps (i) and (ii). Such other any given steps are not particularly limited.

Moreover, the present invention can also provide a substituent-introducing agent for introducing a substituent serving as the above-described protecting group (specifically, a substituent-introducing agent comprising a compound that may act as such substituent), and it can further provide a biomolecule having such substituent (specifically, a protecting biomolecule).

Specifically, a preferred embodiment of the substituent-introducing agent of the present invention is an agent comprising a compound represented by the following formula (I'):

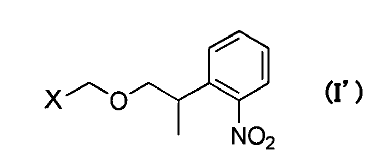

Herein, in the formula (I'), X is not limited, and it preferably represents Cl, I, Br, p-toluenesulfonic acid ester or sulfuric acid ester. Of these, a compound wherein, in the following formula, X is Cl, is more preferable (that is, 2-[(2-nitrophenyl)-chloromethoxy]-propane (NPPOM-Cl)):

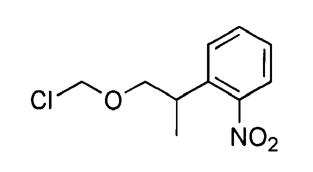

Herein, NPPOM-Cl can be synthesized, for example, according to the method of Example 3 as described later (see scheme 2). Moreover, the formation of a substituent using NPPOM-Cl can also be carried out according to the same above scheme. Furthermore, the synthesis of a compound wherein, in the formula (I'), X is a substance other than Cl, and the formation of a substituent, can also be carried out by referring to the case of NPPOM-Cl, based on common technical knowledge and the like. The substituent formed with the compound represented by the above formula (I') (the substituent derived from the compound) is represented, for example, by a formula as shown below (specifically, a 2-(2-nitrophenyl)propyloxymethyl group).

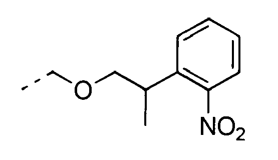

Further, the biomolecule of the present invention includes a biomolecule having a 2-(2-nitrophenyl)propyloxymethyl group (the above formula (I')) (specifically, a biomolecule substituted with the aforementioned group). Specifically, there can be preferably used a biomolecule represented by the following formula (I):

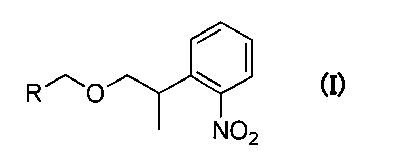

Herein, in the formula (I), R represents any given biomolecule. The biomolecule ® may be either a low-molecular-weight compound (a base, an amino acid, etc.) or a polymer (DNA, RNA, a protein, etc.). It may also be an artificially prepared biocompatible molecule (PNA, etc.). For example, a base is preferable. The type of such base is not particularly limited. Preferred examples of such base include thymine and guanine. When the biomolecule (R) is thymine or guanine, preferred examples of the biomolecule of the present invention include a biomolecule represented by a formula (II) as shown below (specifically, a biomolecule whose thymine has been substituted with (protected by) a 2-(2-nitrophenyl)propyloxymethyl group): 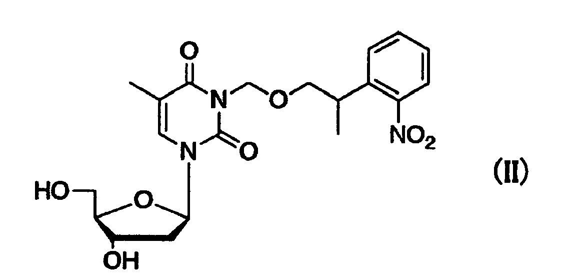 and a biomolecule represented by a formula (III) as shown below (specifically, a biomolecule whose guanine has been substituted with (protected by) a 2-(2-nitrophenyl)propyloxymethyl group):

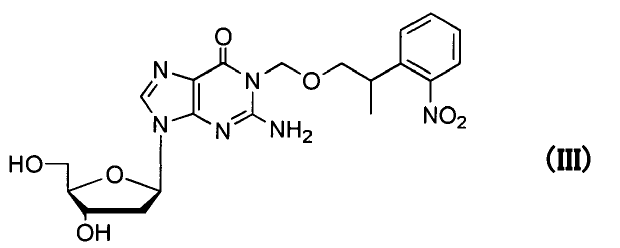

### 3. Genetic recombination method

The genetic recombination method of the present invention comprises a step of binding a DNA fragment obtained by the aforementioned preparation method of the present invention to a host DNA.

Herein, the type of a host DNA is not particularly limited. Any given DNAs, such as various types of vectors (an expression vector, a viral vector, a phage vector, etc.) or the genomic DNAs of various types of organisms, can be used.

As described above, the DNA fragment obtained by the preparation method of the present invention may desirably have a sticky end with a long base length, which cannot be obtained by a restriction enzyme digestion. In this case, the DNA fragment binds to a host DNA via their sticky ends having a long base length. Thus, although a DNA ligase such as a ligase is not particularly used, it becomes possible to bind the DNA fragment to the host DNA with high specificity and with high binding efficiency, only by mixing them. Therefore, the present invention can also provide a genetic recombination method that is excellent in terms of simplicity, economic efficiency and practical utility.

The genetic recombination method of the present invention may also comprise other any given steps, as well as the above-described binding step. Such other any given steps are not particularly limited.

The genetic recombination method of the present invention can be preferably used for the genetic modification of a retrovirus, an adenovirus and the like, which are used for gene therapy or in association with iPS cells. Since such adenovirus and the like have a large genomic DNA, excessive effort and time have been required for the genetic modification of such virus by the conventional method. According to the genetic recombination method of the present invention, the entire genome is divided into portions with a suitable size, and each portion is then amplified by PCR, separately. Thereafter, the amplification products are then ligated to one another via the sticky ends produced by the present invention, so as to easily carry out genetic recombination.

Moreover, the present invention can also provide a genetic recombination kit comprising the DNA fragment obtained by the above-described preparation method of the present invention. The kit may comprise other any given constitutional elements. For example, the kit may comprise various types of vectors such as an expression vector, various types of buffers, sterilized water, a preservative solution, an antiseptic, various types of reaction vessels, manual (instructions) and the like.

Hereinafter, the present invention will be more specifically described in the following examples. However, these examples are not intended to limit the scope of the present invention.

### [Example 1]

### <Development of LACE-PCR (Light-assisted-cohesive-ending PCR) method and application thereof to ARCUT>

### 1. Inhibition of DNA polymerase elongation reaction by T^{NPP}

As shown in Figure 1, in order to confirm the inhibition of an elongation reaction catalyzed by a DNA polymerase (hereinafter, in the present example, simply referred to as a polymerase), a polymerase elongation reaction was examined using C1 and C2 shown in the following Scheme 1. The nucleotide sequences of C1 to C6 shown in the Scheme 1 are shown in SEQ ID NOS: 1 to 6, respectively.

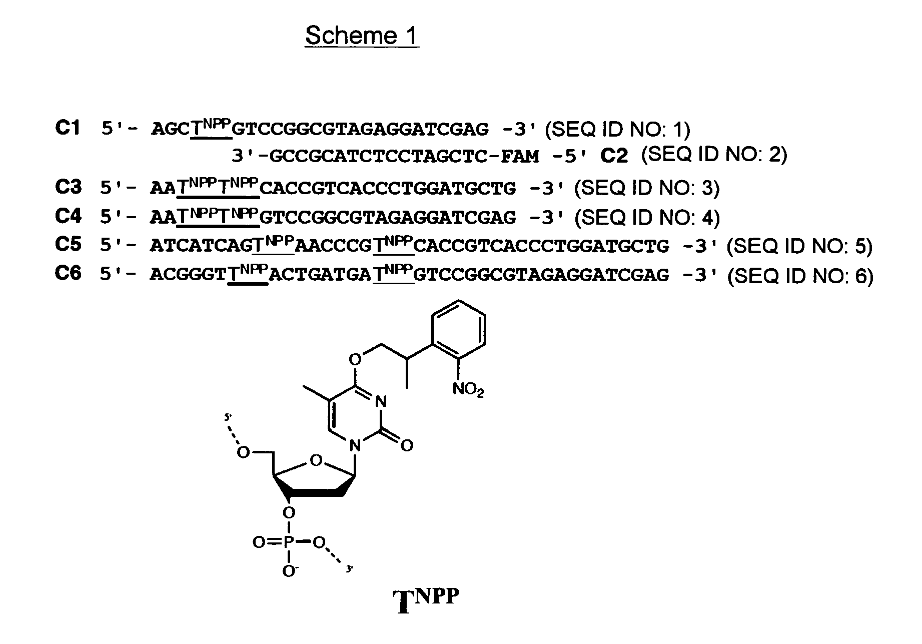

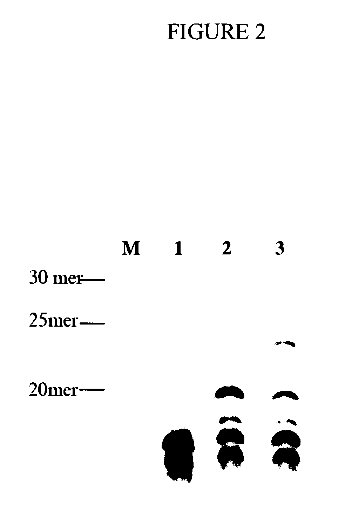

Herein, C1 acts as a template (a template DNA) of C2 used as a PCR primer, and has only one T^{NPP}. The method for synthesizing a primer having such T^{NPP} is as described above. The 5' end of C2 was labeled with FAM. Various types of polymerases were used. Among them, when Ex Taq was used, the clearest results were obtained. The results of the reaction using such Ex Taq are shown in Figure 2. In Figure 2, C1, which had been irradiated with light (UV irradiation) before initiation of the reaction, was used in lane 3. When compared with lane 1, it was found that the elongation reaction in lane 3 progressed to a portion of 24 mer. However, the elongation reaction in lane 2 did not progressed when compared with lane 3, and it could be confirmed that the elongation terminated at a portion of interest (20 mer). With regard to the inhibition of the elongation reaction by C4 and C6 as well, it was confirmed that the elongation reaction terminated at a portion of interest (data not shown). These results yield two conclusions. The first conclusion is that the elongation reaction catalyzed by a polymerase can be terminated at a T^{NPP} of interest. The second conclusion is that the caged protection of T^{NPP} (specifically, protection from the DNA polymerase elongation reaction by an NPP group (inhibition of the elongation reaction)) is efficiently deprotected by UV irradiation. It was also found that, in order to terminate the elongation reaction, it is sufficient to contain only one T^{NPP}.

It has previously been known that the elongation reaction catalyzed by a polymerase is suppressed by a TT dimer. It has been reported that, utilizing such phenomenon, the elongation reaction is inhibited by a template in which two modified bases are aligned, and that the elongation reaction is then promoted by dissociating the one modified base by light irradiation (Tang, X. J. et al., (2005) Photoregulation of DNA polymerase I (Klenow) with caged fluorescent oligodeoxynucleotides. Bioorg. Med. Chem. Lett., 15, 5303-5306). Thus, the present inventor considered that the inhibition efficiency would be enhanced by introducing two T^{NPP} residues, and introduced such two T^{NPP} residues into the C3 primer.

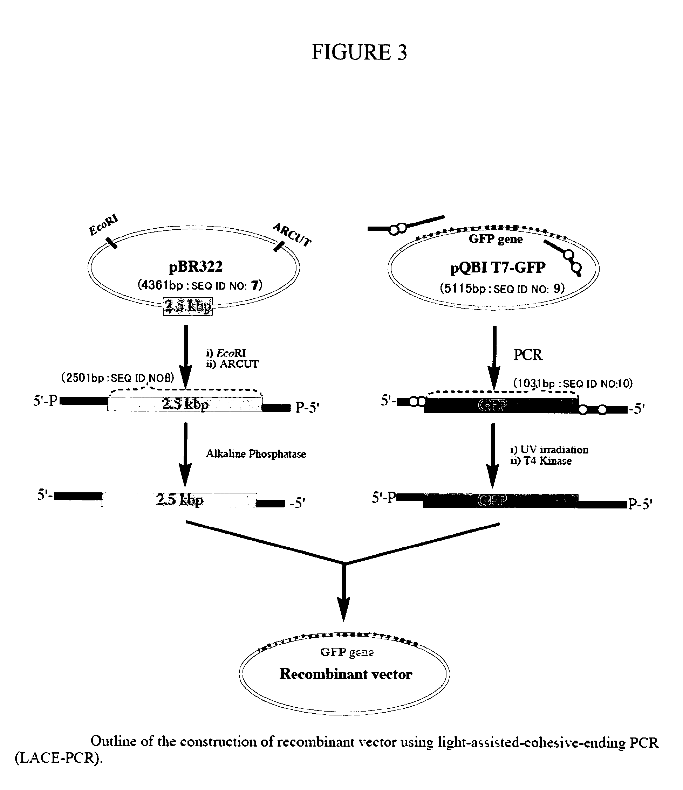

According to the method of the present invention, it became possible to produce a DNA fragment having a desired 5' protruding end. Thus, various insert DNA fragments (inserts) were actually prepared according to PCR using desired synthetic primers, and the thus prepared inserts were each inserted into a vector according to a common method. In the present example, a summary of a method using a vector cleaved by restriction enzyme (*Eco*RI) or ARCUT is shown in Figure 3. It is to be noted that, for ARCUT (an artificial restriction DNA cutter), "Yamamoto, Y. et al., Chem. Bio. Chem., vol. 7, pp. 673-677, 2006" can be referred.

### 2. Insertion into single cleavage site by EcoRI

### 2-1. Designing and synthesis of EcoRI LACE primer

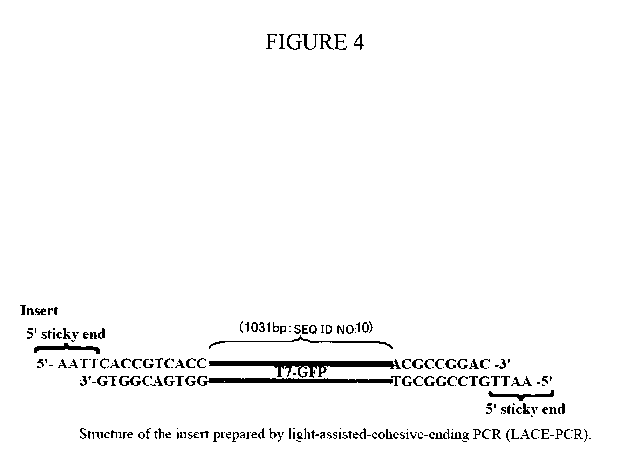

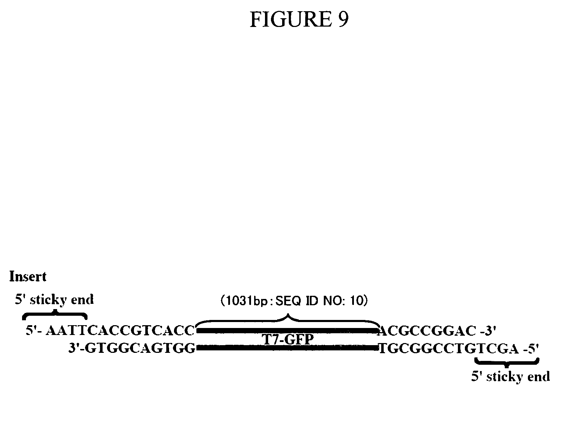

In order to cleave a vector (pUC18) by *Eco*RI and then insert the prepared insert into the cleaved portion, PCR primers C3 and C4, as shown in the Scheme 1, were synthesized. The primers C3 and C4 were designed, so that they were able to amplify a nucleotide sequence region (121-1170 bp) comprising both the GFP gene of pQBI T7-GFP (Qbiogene; 5115 bp (SEQ ID NO: 9)) and a T7 promoter sequence, and so that the insert obtained as an amplification product had a 5' protruding end complementary to the *EcoRI* cleavage fragment (Figure 4). In the sequence of the insert shown in Figure 4, the nucleotide sequence portion indicated as T7-GFP (comprising the GFP gene and the T7 promoter sequence; total 1031 bp; consisting of bases 131-1161 in the nucleotide sequence shown in SEQ ID NO: 9) is shown in SEQ ID NO: 10 (the same applies to Figures 9, 14b and 16b). As stated above, in order to enhance inhibition efficiency, two T^{NPP} residues were introduced into each primer. Hereinafter, the primer used in LACE-PCR was referred to as a LACE primer.

### 2-2. Insertion of LACE-PCR product into vector (production of recombinant vector)

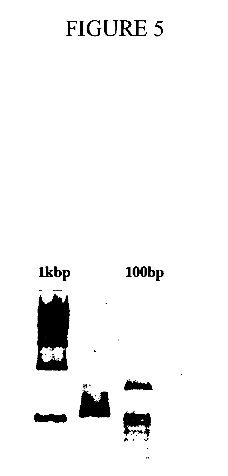

A vector (pUC18) was prepared by a known general method. In order to prevent the autocyclization of a plasmid DNA, dephosphorylation was carried out with phosphatase. When an insert was prepared, KOD-Plus with high precision was used as a polymerase for PCR. Looking at the product obtained after PCR shown in Figure 5, a band was observed at 1 kbp, and thus it was found that PCR was carried out with no problems. Thereafter, the protecting group was dissociated by light irradiation, and phosphorylation was then carried out with a kinase. If deprotection was not carried out at this stage, there was the possibility that the kinase could not work well. In a case in which a primer whose 5' end had previously been phosphorylated was used, the aforementioned operation could be omitted. The prepared vector was ligated to the insert, and was then transformed. As a result, colonies of transformant were observed. Thus, positive colonies were selected by colony PCR.

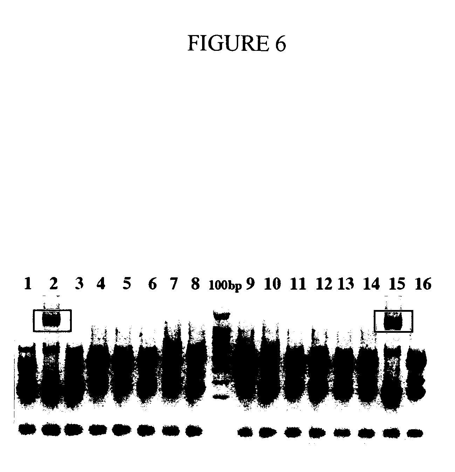

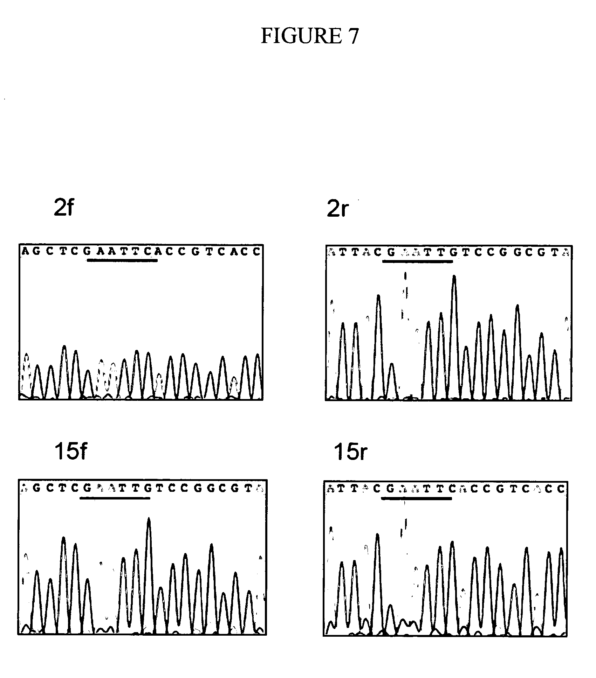

In Figure 6, the plasmid DNAs of positive colonies in lane 2 and lane 15 were extracted, and the nucleotide sequences thereof were read. As a result, it could be confirmed that an insert was inserted therein in a form of interest (Figure 7). When such insert was introduced into a single cleavage site, there were two ways of introducing the insert. That is, it was found that the insert was inserted in a desired direction in the case of lane 2, and that the insert was inserted in a reverse direction in the case of lane 15. In addition, it was also found that the sequence GAATTG was inserted on one side, and thus that one cleavage site recognized by *Eco*RI was broken. This method is **characterized in that** it is able to break a restriction site, and it is excellent in terms of usefulness and practical utility.

Thus, it was demonstrated that this method is able to precisely insert the insert prepared by LACE-PCR into a vector, so as to produce a recombinant vector.

### 3. Evaluation of LACE primer by HPLC

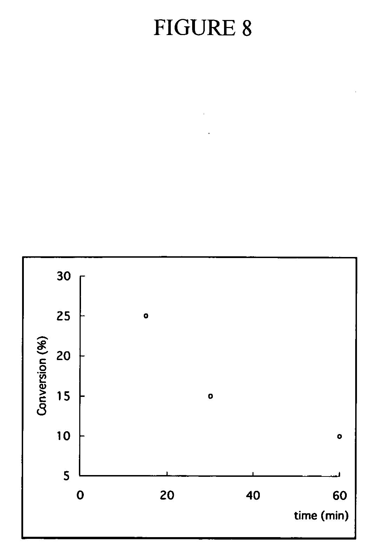

In order to evaluate the level of deprotection of a caged compound (a DNA having a base (T) protected by an NPP group) by UV irradiation, UV was applied to C3 and C4, and these primers were then analyzed by RP-HPLC. The results are shown in Figure 8. In this analysis, since the detection wavelength was set at 260 nm. Thus, there was the possibility that the abundance ratio and the peak area ratio would be shifted because a compound with NPP as a protecting group and a compound without such NPP had different absorption coefficients. However, since the total peak area was not different so much before and after UV irradiation, the ratio was calculated, considering that such difference in absorption coefficients was negligible. From the graph shown in Figure 8, it was found that 90% or more of NPP was deprotected by UV irradiation for 1 hour. This result shows that a sufficient amount of caged deprotection was carried out by UV irradiation for 1 hour. If it is assumed that 90% of two deprotections was completed, 80% thereof was a product of interest.

Since PCR conditions were relatively radical (heating to 90°C, etc.), there was possibility that NPP was dissociated by heat. Hence, C3 was incubated under the same conditions as PCR conditions, and it was then analyzed by HPLC. As a result, the percentage of compounds from which two NPPs acting as protecting groups were dissociated was 7.7%, the percentage of compounds from which one NPP was dissociated was 18.9%, and the percentage of compounds from which no NPP was dissociated was 73.4%. That is to say, the percentage of compounds, from which NPP on the 3' side was not dissociated but remained, was considered to be approximately 85%. Since two primers were used in PCR, the percentage, in which a base at a position of interest was caged, was 70%.

Based on the aforementioned two factors, when NPP protection was used, the percentage of obtaining a PCR product having a 5' protruding end of interest was considered to be approximately 50% to 60%.

### 4. Insertion of LACE-PCR product into two cleavage sites by EcoRI-HindIII

### 4-1. Designing and synthesis of EcoRI-HindIII LACE primer

As described above, genetic recombination operations were successfully carried out in the case of a system of one cleavage site. Next, the inventor made a second attempt to insert LACE-PCR products into a system of two cleavage sites. Basically, an experiment was carried out by the same method as that applied to the system of one cleavage site. C1 was used as a LACE primer, and it was designed to have a *Hind*III protruding end. In addition, in order to obtain an *Eco*RI protruding end, C3 was used. At this time, the LACE primer had only one caged protection. Thereby, whether or not a polymerase elongation reaction was terminated by one caged protection was simultaneously examined under PCR conditions. If the reaction is successfully carried out as intended, then the insert as shown in Figure 9 is obtained.

### 4-2. Insertion of LACE-PCR product into vector (production of recombinant vector)

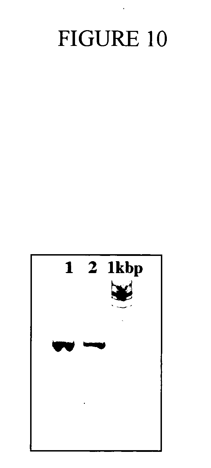

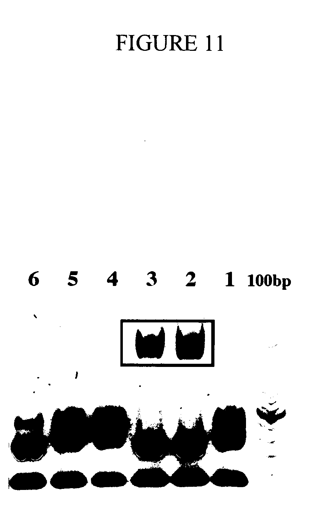

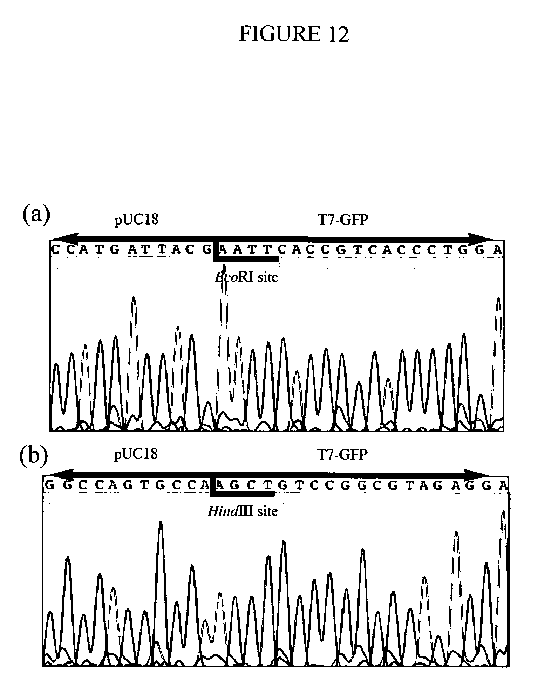

Basic procedures were the same as those described in Section 2 above. That is, pUC18 was reacted with *Hind*III at 37°C for 4 hours, and it was further reacted with *Eco*RI at 37°C for 16 hours, so as to prepare a vector having two cleavage sites. C1 and C3 were used as LACE-PCR primers, so as to prepare an insert (Figure 10, lane 2). In addition, it could be confirmed from lane 1 that the prepared insert was not apparently damaged (for example, was not disconnected) by UV irradiation. The prepared insert was ligated to the vector, so that transformation was carried out. As a result, only 6 colonies of transformants were observed, and the two colonies thereof were found to be positive by colony PCR (lane 2 and lane 3 in Figure 11). The plasmid DNAs of such positive transformants were extracted, and the nucleotide sequences thereof were read. As a result, it was found that the insert could be inserted in a form of interest (Figure 12). Thereby, it could be confirmed that the prepared insert was sufficiently applicable to the system of two cleavage sites. Moreover, it was also demonstrated that only one caged protection is sufficient under these PCR conditions.

### 5. Insertion of LACE-PCR product into one cleavage site by ARCUT

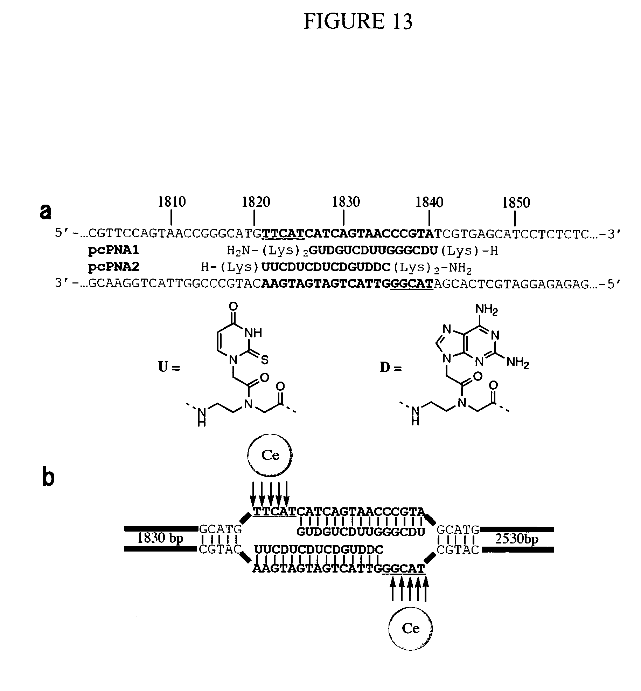

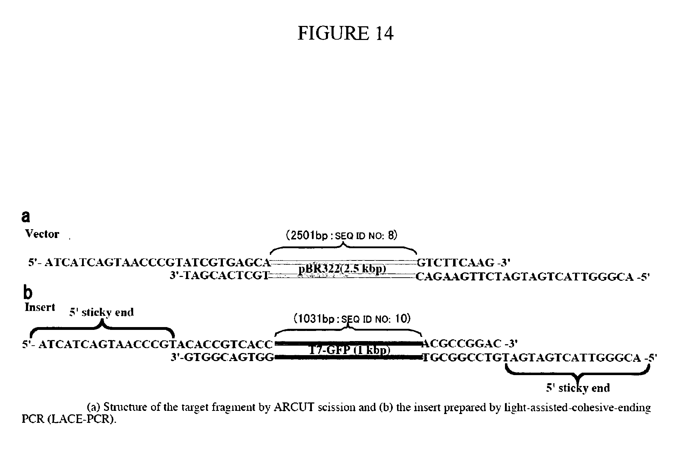

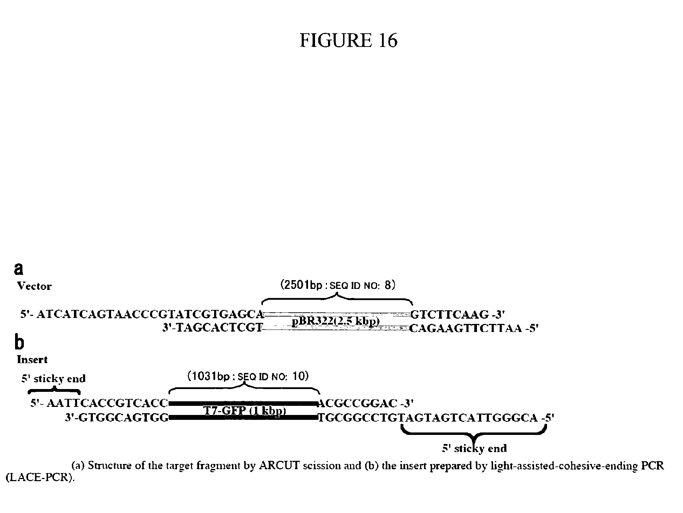

As described above, with regard to systems in which a vector was cleaved by a natural restriction enzyme, the insert prepared by LACE-PCR was successfully introduced into both the system of one cleavage site and the system of two cleavage sites. The greatest characteristic of this method is that a protruding end having any given sequence can be produced. Thus, the present inventor has thought of the possibility of directly ligating the insert to a protruding end generated after ARCUT cleavage, which does not exist in the nature. The inventor has attempted to directly insert the insert into a system in which pBR322 (Takara Bio Inc.; 4361 bp (SEQ ID NO: 7): GenBank Accession No. J01749) was cleaved by ARCUT at one cleavage site. As shown in Figure 13a, the target cleavage site by ARCUT was determined around 1830 bp of the pBR322. The used pcPNA (pseudo-complementary peptide nucleic acid) is also shown in Figure 13 a. The cleavage of the vector by ARCUT occurs at any of the sites indicated with the arrows shown in Figure 13b. This time, a vector fragment (Figure 14a) generated as a result of the cleavage occurring at the site with the blue arrow (the second arrow from the right among the 5 arrows on both strands shown in Figure 13b) was used as a target. With regard to the vector fragment shown in Figure 14a, the nucleotide sequence of a portion indicated with pBR322 in the center (total 2501 bp: consisting of bases 1850-4350 in the nucleotide sequence shown in SEQ ID NO: 7) is shown in SEQ ID NO: 8 (the same applied to Figure 16a). Thus, the C5 and C6 PCR primers in the above Scheme 1 were designed to produce an insert fragment having a protruding end shown in Figure 14b. Using pQBI T7-GFP as a template, PCR and light irradiation were carried out by the same method as that in the Section 2-1 above, and as a result, a desired insert fragment (Figure 14b) was synthesized and purified. An extremely important point for designing primers will be described below. Since the protruding end of ARCUT is extremely long, primers used in LACE-PCR also become long. The two primers comprise sequences complementary to each other, and thus they easily form a primer dimer. Hence, as with C5 and C6, T^{NPP} has been introduced into such a portion complementary to each other to inhibit the interaction between the primers, and thus, the primers were designed so as not to form a primer dimer.

### 5-2. Single-site cleavage of pBR322 by ARCUT and purification of cleavage fragment

A fragment obtained by cleaving supercoiled pBR322 DNA by ARCUT was purified using gel, so as to obtain a linearized fragment. Thereafter, an excessive amount of oligo DNA complementary to PNA was added thereto to remove PNA, and a phosphatase treatment was then performed for dephosphorylation.

### 5-3. Insertion of LACE-PCR product into vector (production of recombinant vector)

It was confirmed that the insert was amplified by LACE-PCR as designed. Thus, after completion of the caged deprotection, the resultant was treated with kinase, and insertion of the product into the vector linearized by ARCUT was then attempted. However, in conclusion, this system was not successful at all. The main reason is considered that, since the protruding end is long, autocyclization occurs more easily than a system cleaved only at a single site by restriction enzyme. The amount of the insert was increased from 50 equivalents to 200 equivalents with respect to the amount of the vector, and ligation was carried out. However, no positive colonies could be obtained by colony PCR in all the cases. Moreover, even in a case in which *Escherichia coli* was transformed without ligation reactions, colonies were increased. As a result, it was considered that the insert could be sufficiently inserted into this system even without ligation reactions.

### 6. Insertion of LACE-PCR product into two cleavage sites by EcoRI-ARCUT

### 6-1. Designing of EcoRI-ARCUT LACE primer

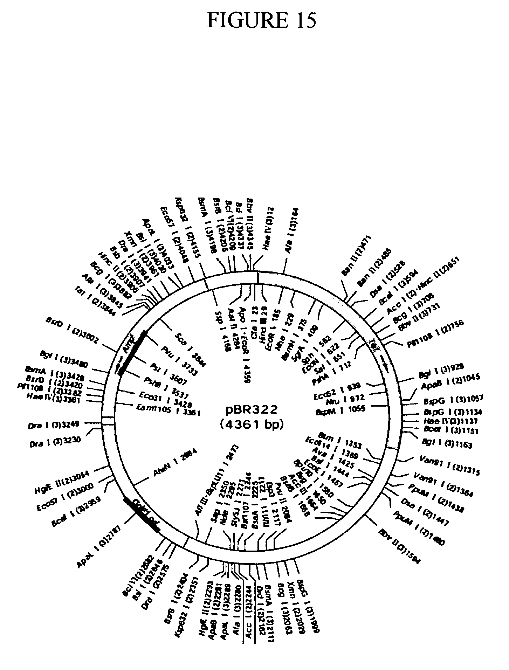

Two fragments, 1830 bp and 2530 bp, can be obtained by cleaving the pBR322 linearized by *Eco*RI at a site around 1830bp. Of the two fragments, the 2530 bp fragment has a drug resistance gene (Amp) and a replication origin (ori), and thus it acts as a vector (Figure 15). Hence, using the previously synthesized C3 and C6 as LACE primers, a fragment having a protruding end as shown in Figure 16b was produced, and the insert was then inserted into an ARCUT cleavage fragment as shown in Figure 16a.

### 6-2. Two-site cleavage of pBR322 by EcoRI and ARCUT, and purification of cleavage fragment

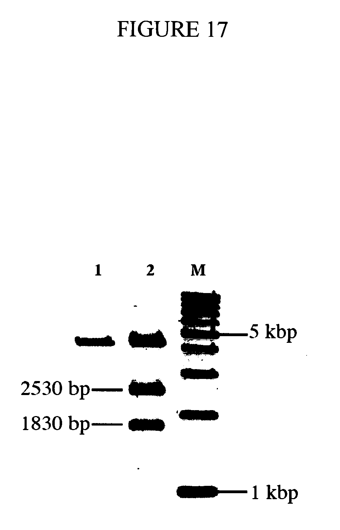

The pBR322 linearized by *Eco*RI was further cleaved by ARCUT, and the resultant was then purified in the same manner as in the Section 4-2 above. Figure 17 shows the results of electrophoresis performed after the cleavage of pBR322 (lane 1: cleaved only by *Eco*RI*;* lane 2: cleaved by *both Eco*RI and ARCUT).

### 6-3. Insertion of LACE-PCR product into vector (production of recombinant vector)

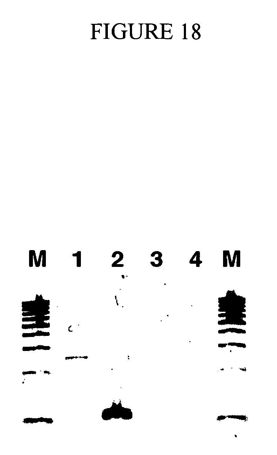

It was confirmed that the insert was amplified by LACE-PCR as designed. Thus, after completion of the caged deprotection, the resultant was treated with kinase. Thereafter, the vector prepared in the Section 5-2 above was mixed with the insert at a mixing ratio of 1 : 40 to ligate them, and the obtained ligate was then introduced into DH5α so as to transform it. Since generation of colonies was confirmed, colony PCR was carried out in the same manner as that described above, and the plasmid DNA of positive colonies was extracted. The plasmid DNA was cleaved at a single site by *Eco*RI*,* so that the size of the extracted plasmid DNA was confirmed (Figure 18, lane 4).

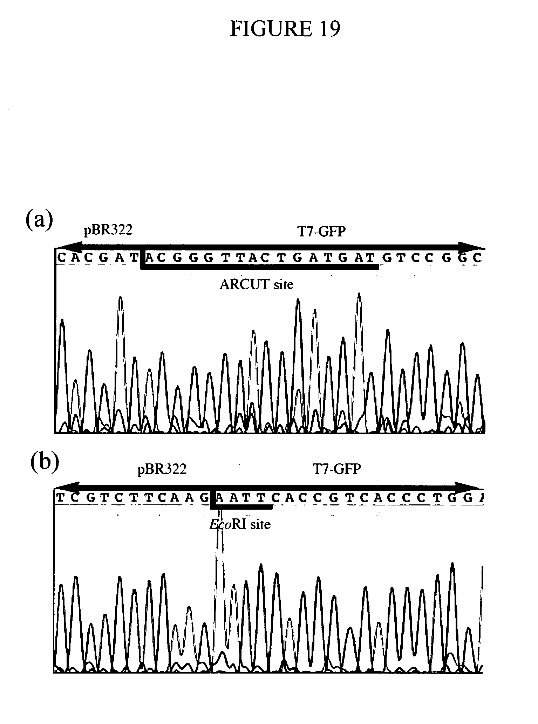

The nucleotide sequence of the plasmid DNA was determined by the same method as that described above. As a result, as shown in Figure 19, it was found that a portion cleaved by *Eco*RI and a portion cleaved by ARCUT could be ligated at positions of interest.

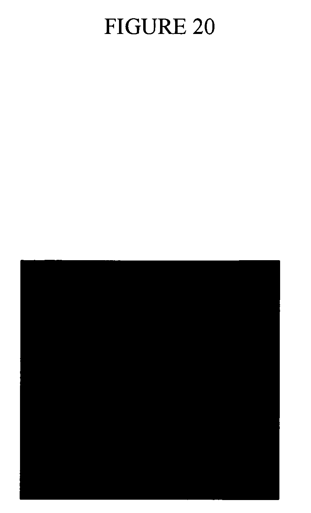

Furthermore, the present plasmid was introduced into BL21-Gold (DE3) having a T7 RNA polymerase so as to transform it. As a result, it could be confirmed that GFP was expressed (Figure 20).

### 7. Conclusion and consideration

To sum up the present examples, when a caged DNA was used as a template, polymerase was not able to promote an elongation reaction (a complementary strand synthetic reaction) after a base protected by a protecting group, and any given 5' end protruding structure was formed. Since such caged protection could be easily deprotected by UV irradiation, this technique was applied to genetic recombination technology. That is, PCR was carried out using a caged DNA as a primer, so that a 5' protruding end could be successfully prepared (LACE-PCR). The insert prepared by LACE-PCR was successfully inserted into a vector subjected to a restriction enzyme digestion and a vector digested by ARCUT. Thereby, the inventor succeeded in performing ligation on an ARCUT inserted fragment at a position of interest without using Oligojoint. As a result of the establishment of this technique, this technique was considered to be applied, for example, to genetic engineering using ARCUT. In recent years, the chemical synthesis of bacterial genome has been succeeded. In this chemical synthesis as well, one key point was production of a specific protruding end. It was considered that gene synthesis, DNA-overhang cloning (DOC) and the like could be accomplished by applying the method of the present invention.

### [Example 2]

### <Genetic recombination that utilizes LACE-PCR method without using ligase (Ligation-Independent Cloning)>

### 1. Production of GFP-BFP gene that involves insertion into plasmid comprising GFP gene

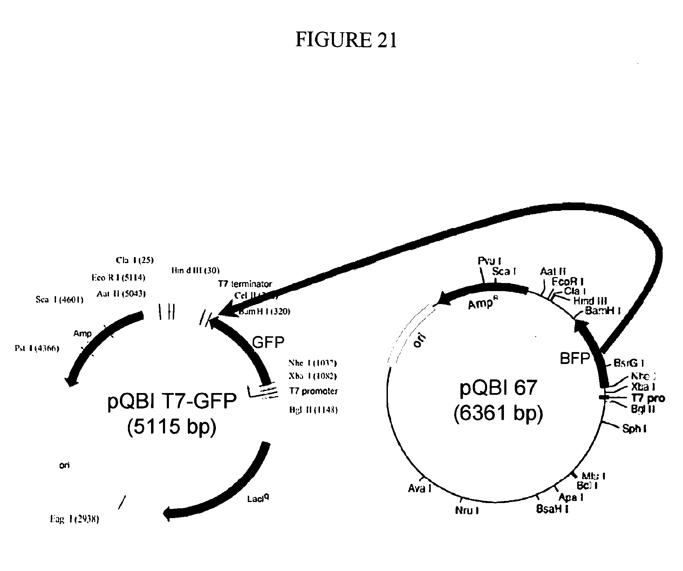

It has been known that, when two fragments having relatively long sticky ends are used, transformation can be carried out by utilizing the repair mechanism of *Escherichia coli* without ligation. An entire-length of a pQBI T7-GFP vector (Qbiogene; 5115 bp (SEQ ID NO: 9); Figure 21, left) having a long sticky end consisting of approximately 10 bases, which had been amplified by LACE-PCR, was allowed to bind to a BFP fragment in a pQBI 67 vector (Qbiogene; 6361 bp; Figure 21, right), which had also been amplified by LACE-PCR, thereby constructing a novel vector encoding a GFP-BFP fusion protein (see Figure 21).

### 2. Designing and synthesis of LACE primers

### 2-1. Primers used in amplification of pQBI-T7 GFP vector

In order to amplify an entire-length of a pQBI-T7 GFP vector, the following PCR primers, C7 and C8, were synthesized.

| | |
|---|---|
| C7 | 5'-pACCGCCACC**T^{NPP}**CCGTTGTACAGTTCATCCATGCC-3' (SEQ ID NO: 11) |
| C8 | 5'-pAGGATCCGGC**T^{NPP}**GCTAACAAAGCCCGAAAGGAAGC-3 (SEQ ID NO: 12) |

C7 and C8 are primers for amplifying the entire nucl obtained by removing bases 314 to 328 from the pQBI T7-GFP vector. Since me mus removed region comprises the step codon of the GFP gene, if a DNA fragment comprising a foreign protein gene is inserted, a fusion protein with GFP is translated (generated). To the 5' end side from the T^{NPP} of the primer that would become a sticky end as a result of LACE-PCR, 9 bases encoding a glycine linker (C7) or 10 bases identical to bases 315 to 324 of the pQBI T7-GFP vector (C8) was added.

### 2-2. Primers used in amplification of BFP gene

In order to amplify a BFP gene encoded by a pQBI 67 vector by LACE-PCR, the following PCR primers, C9 and C 10, were synthesized.

| | |
|---|---|
| C9 | 5'-pAGCCGGATCC**T^{NPP}**CAGTTGTACAGTTCATCCATGCC-3' (13) |
| C10 | 5'-pAGGTGGCGG**T^{NPP}**GGCAAAGGAGAAGAACTCTTCACTGG-3' (14) |

C9 and C10 are primers for amplifying a nucleotide sequence region (701 bp; SEQ ID NO: 15) encoding a main body of BFP consisting of bases 335 to 1035 of the pQBI 67 vector. To the 5' end side from the T^{NPP} of the primer that would become a sticky end as a result of LACE-PCR, 9 bases encoding a glycine linker (C10) or 10 bases identical to bases 315 to 324 of the pQBI T7-GFP vector (C9) was added.

### 2-3. LACE-PCR

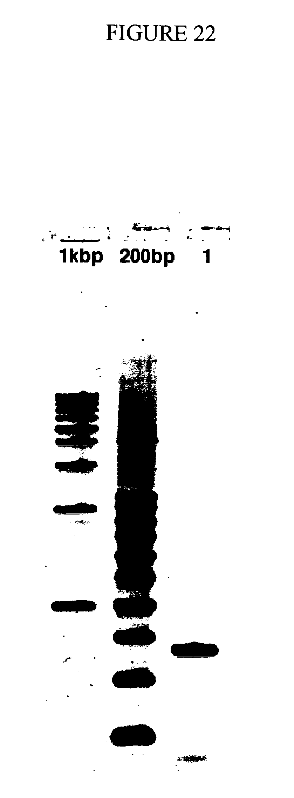

LACE-PCR was carried out using the above-described primers. For amplification of the vector fragments C7 and C8, commercially available *Pfu* ultra was used. For amplification of the BFP inserts C9 and C10, *Pfu* turbo was used as polymerase in PCR. After completion of the PCR, the PCR product was digested with Dpn I for digesting a methylated DNA at 37°C overnight, and the amplified fragment was simply purified using a commercially available kit, so as to obtain an aqueous solution (TE buffer) of each fragment. Subsequently, the NPP protecting group was deprotected by UV irradiation. The results obtained by subjecting the amplified fragment to agarose electrophoresis are shown in Figure 22. In the case of an amplified fragment supplied to lane 1, a band was observed between a 600-bp fragment and a 800-bp fragment on a ladder of 200-bp units, and thus it was confirmed that a 710-bp fragment was amplified as designed.

### 2-4. Transformation of Escherichia coli with LACE-PCR product

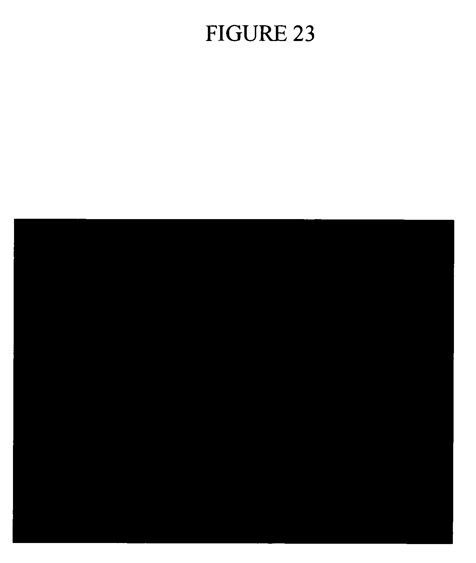

A solution corresponding to 50 ng of a vector fragment and a solution corresponding to 24 ng of a BFP insert (approximately 3 equivalents relative to mole; total amount: 2 µL) were mixed to each other, and the mixed solution was then incubated at 37°C for 1 hour. Thereafter, the reaction solution was added to a solution of JM109 *Escherichia coli* strain without a ligation reaction, so that the *Escherichia coli* was transformed. This *Escherichia coli* solution was cultured on a plate containing 50 µg of carbenicillin overnight. As a result, 6 colonies were obtained. Colony PCR was carried out, and as a result, it was confirmed that 5 colonies were recombinant bodies of interest. Each colony was subjected to liquid culture, and its plasmid was extracted. Thereafter, a BL21(DE3) strain was transformed with each plasmid. As a result of culture on a plate containing 50 µg of carbenicillin, the generated colony emitted dark green fluorescence. Figure 23 shows the color of the fluorescence of this *Escherichia coli* strain. The two letters "UT" indicate the fluorescence of the recombinant body produced in the present example. The color of the fluorescence was different from either the letter "L" having only the GFP gene or the letters "ACE" having only the BFP gene.

### 2-5. Confirmation of construction of recombinant vector as designed

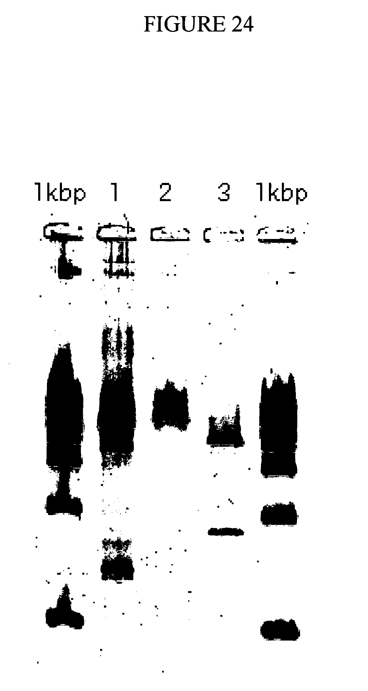

The extracted plasmid was cleaved by restriction enzymes, and whether it had a correct length was then confirmed. The results of agarose gel electrophoresis are shown in Figure 24. The extracted plasmid (lane 2), which had been converted to a straight chain by a digestion with Eco RI, clearly had a mobility on electrophoresis that was lower than that of a vector fragment amplified by LACE-PCR (lane 1; 5125 bp). Thus, it was found that the BFP fragment was correctly inserted. This result could also be confirmed by the fact that the predicted 1862-bp and 3975-bp bands were clearly observed also in lane 3 involving two-site cleavage by Eco RI and Nhe I.

### 2-6. Confirmation of protein expressed by recombinant vector

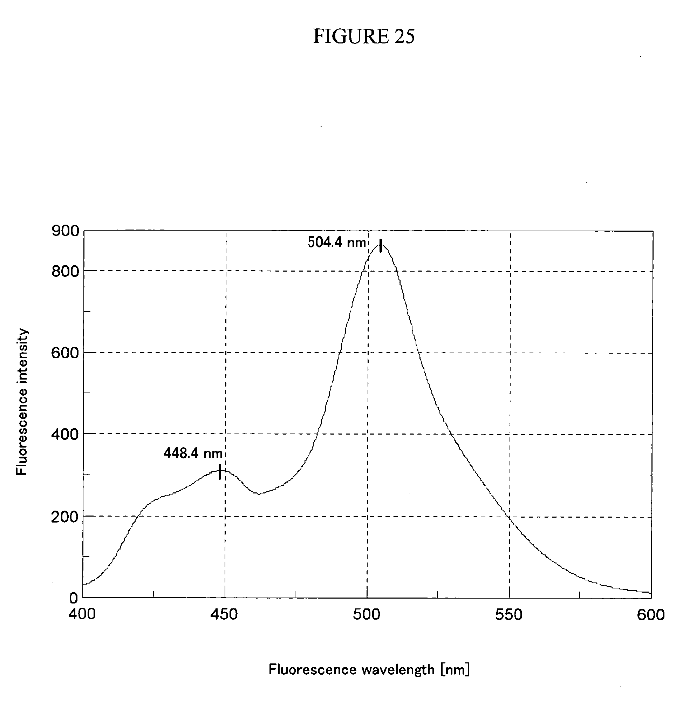

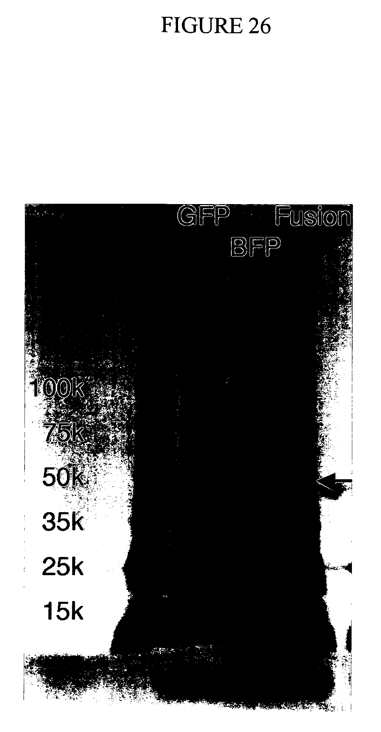

In order to confirm that a protein expressed by the recombinant vector is correctly a GFP-BFP fusion protein, the transformed *Escherichia coli* was subjected to liquid culture, and was then subjected to a bacteriolytic treatment. The fluorescence of the bacteriolytic solution was measured. As a result, two fluorescence peaks, 448 nm derived from BFP and 504 nm derived from GFP, were confirmed (Figure 25). In addition, the bacteriolytic solution was analyzed by SDS-PAGE. As a result, it was confirmed that a novel approximately 50 kDa protein (a GFP-BFP fusion protein; Fusion), which differed from either BFP (26.9 kDa) or GFP (26.9 kDa), was strongly expressed (Figure 26).

### [Example 3]

### <Synthesis of thymine protected by 2-(2-nitrophenyl)propyloxymethyl group and amidite monomer>

An amidite monomer to be introduced into thymine protected by a 2-(2-nitrophenyl)propyloxymethyl group was synthesized according to the following Scheme 2.

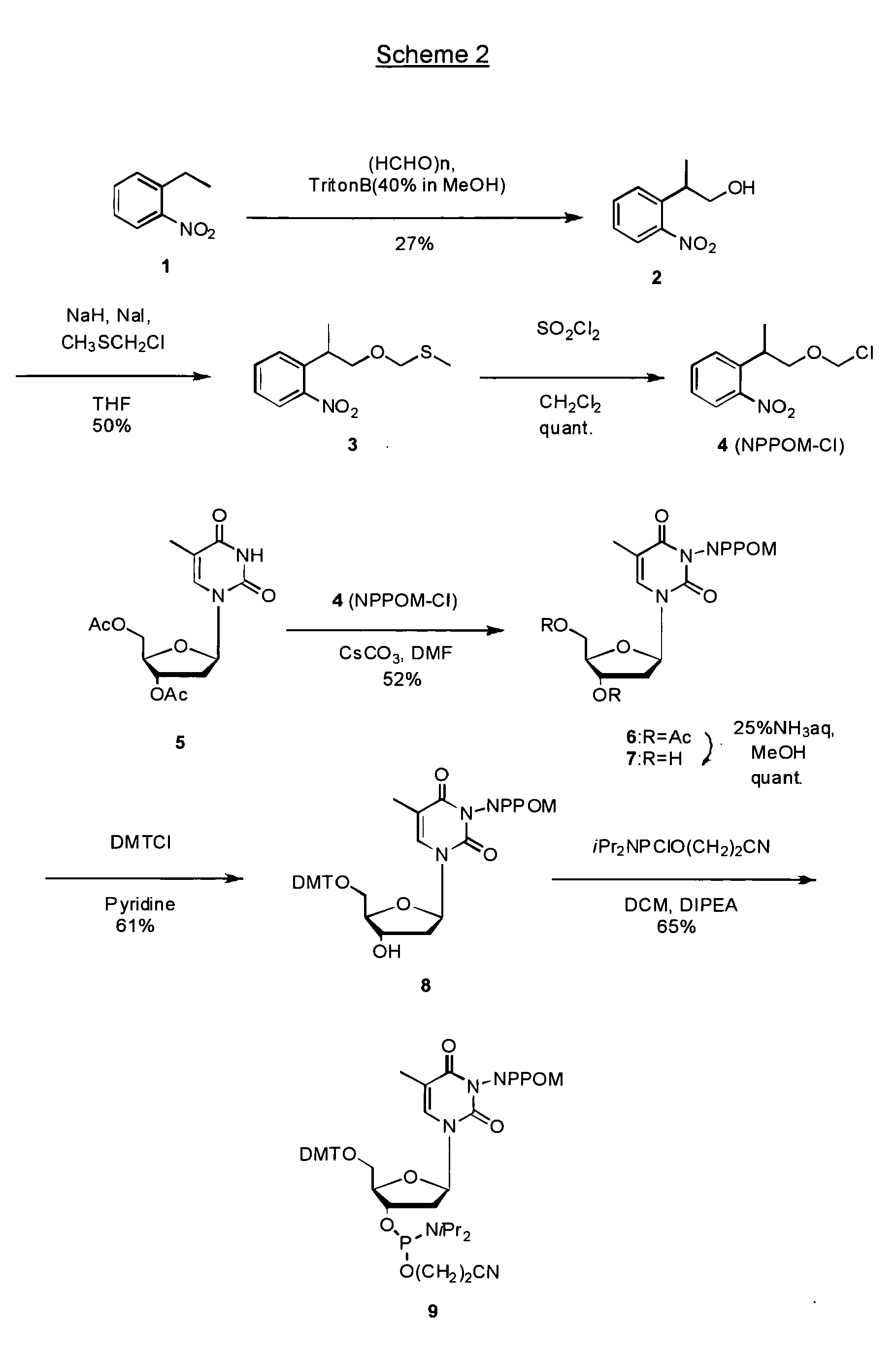

Compounds (Compounds 2-9) shown in the Scheme 2 were specifically synthesized as follows.

### Synthesis of 2-(2-nitrophenyl)propanol (Compound 2)

Compound 1 (2-ethylnitrobenzene) (6.0 g, 40 mmol) and (HCHO)ₙ (1.5 g, 40 mmol) were added into TritonB (40% MeOH solution) (17 g), and the obtained mixture was then heated under reflux for 7 hours. Thereafter, the solvent was distilled away, and the resultant was then re-dissolved in ethyl acetate. The organic layer was washed with 5% hydrochloric acid. The organic layer was dried over sodium sulfate, and was then purified by silica gel chromatography using hexane : ethyl acetate (3 : 1) as a developing solvent, so as to obtain 1.95 g of a reddish violet oily product of interest (Compound 2) (10.7 mmol, yield: 27%).
¹H NMR (CDCl₃) δ= 1.34 (3H, d, CH₃), 3.50-3.58 (1H, m, CH), 3.77-3.86 (2H, m, CH₂), 7.36 (1H, t, Ar-H), 7.50 (1H, d, Ar-H), 7.58 (1H, t, Ar-H), 7.76 (1H, d, Ar-H)

### Synthesis of 2-[(2-nitrophenyl)-(methylthio)methoxy]-propane (Compound 3)

Compound 2 (1.95 g, 10.7 mmol) was dissolved in 20 mL of THF, and the obtained solution was then cooled at 0°C in a nitrogen atmosphere. While stirring, sodium hydride (60%) (0.6 g) was added to the reaction solution. The obtained mixture was stirred for 45 minutes, and sodium iodide (1.62 g, 10.7 mmol) was then added to the reaction solution. Subsequently, chloromethyl methyl sulfide (ClCH₂SCH₃; 1.29 g, 13.35 mmol) was added thereto. The obtained mixture was stirred under cooling on ice for 4 hours, and the temperature of the reaction solution was then returned to room temperature. Thereafter, the solvent was distilled away. 30 mL of water was added to the residue, and was then extracted with ether. The organic layer was dried over sodium sulfate, and was then purified by silica gel chromatography using dichloromethane as a developing solvent, so as to obtain 1.32 g of a brown oily product of interest (Compound 3) (5.43 mmol, yield: 50%).
¹H NMR (CDCl₃) δ=1.36 (3H, d, CH₃), 2.00 (3H, s, CH₃), 3.63-3.76 (3H, m, CH, CH₂), 4.58 (2H, d, CH₂), 7.34 (1H, t, Ar-H), 7.52 (2H, m, Ar-H), 7.75 (1H, d, Ar-H)

### Synthesis of 2-[(2-nitrophenyl)-chloromethoxy]-propane (Compound 4)

Compound 3 (1.32 g, 5.43 mmol) was dissolved in 20 mL of dichloromethane. While the obtained solution was stirred at 0°C in a nitrogen atmosphere, SO₂Cl₂ (0.94 g, 6.96 mmol) was added to the solution. The obtained mixture was stirred under cooling on ice for 4 hours, and the temperature of the reaction solution was then returned to room temperature. Thereafter, an excessive amount of SO₂Cl₂ was distilled away under reduced pressure. The reaction was quantitative. The obtained compound (Compound 4) was directly used in the subsequent reaction without purification.
¹H NMR (CDCl₃) δ=1.37 (3H, d, CH₃), 3.66 (1H, m, CH), 3.81 (2H, m, CH₂), 5.43 (2H, q, CH₂), 7.36 (1H, t, Ar-H), 7.48 (1H, d, Ar-H), 7.57 (1H, t, Ar-H), 7.76 (1H,d, Ar-H)

### Synthesis of thymidine acetate (Compound 5)

Thymidine (2.93 g, 12.1 mmol) and DMAP (40 mg, 0.327 mmol) were dissolved in 60 mL of acetic anhydride in a nitrogen atmosphere, and 30 mL of dry pyridine was then added to the solution, followed by stirring at room temperature overnight. Thereafter, pyridine and acetic anhydride were distilled away under reduced pressure, and 100 mL of chloroform was then added to the residue. The organic layer was washed with 100 mL of water. The organic layer was dried over anhydrous sodium sulfate, and was then purified by silica gel chromatography using hexane : ethyl acetate (1 : 3) as a developing solvent, so as to obtain 2.82 g of a product of interest (Compound 5) (8.64 mmol, yield: 71%).
¹H NMR (CDCl₃) δ=1.94 (3H, s, CH₃), 2.10-2.22 (8H, m, 2×CH₃, CH₂), 2.46 (1H, m, CH), 4.36 (2H, dd, CH₂), 5.22 (1H, d, CH), 6.32 (1H, q, CH), 7.29 (1H, s, CH), 8.65 (1H, s, NH)

### Synthesis of 3-N-[2-(2-nitrophenyl)-propyloxymethyl]thymidine acetate (Compound 6)

Compound 5 was dissolved in 20 mL of DMF, and cesium carbonate was then added to the solution, followed by stirring at room temperature for 1 hour. Subsequently, the reaction solution was cooled on ice, and Compound 4 (1.26 g, 5.47 mmol) dissolved in DMF was then added dropwise thereto. The solvent was distilled away under reduced pressure, and ethyl acetate was then added to the residue. The organic layer was washed with 30 mL of water twice, and then with a saturated sodium bicarbonate solution once. It was then dried over sodium sulfate. The solvent was distilled away under reduced pressure, and the residue was then purified by silica gel chromatography using hexane : ethyl acetate (1 : 2) as a developing solvent, so as to obtain 1.50 g of a product of interest (Compound 6) (2.89 mmol, yield: 52%).
¹H NMR (CDCl₃) δ=1.31 (3H, dd, CH₃), 1.93 (3H, d, CH₃), 2.11 (8H, m, 2×CH₃, CH₂), 3.57 (1H, m, CH), 3.74-3.88 (2H, m, CH₂), 4.25 (1H, s, CH), 4.34 (2H, m, CH₂), 5.21 (1H, d, CH), 5.36 (2H, m, CH₂), 7.28 (1H, s, CH), 7.31 (1H, t, Ar-H), 7.46 (1H, d, Ar-H), 7.51 (1H, t, Ar-H), 7.71 (1H, t, Ar-H)

### Synthesis of 3-N-[2-(2-nitrophenyl)-propyloxymethyl]thymidine (Compound 7)

Compound 6 (1.50 g, 2.89 mmol) was dissolved in 20 mL of methanol, and 8 mL of concentrated ammonia water was then added to the solution, followed by stirring at room temperature for 1 hour. Thereafter, the solvent was distilled away, so as to quantitatively obtain a product of interest (Compound 7)
¹H NMR (CDCl₃) δ=1.31 (3H,d, CH₃), 1.90 (3H, d, CH₃), 2.31-2.42 (2H, m, CH₂), 3.55-3.65 (1H, m, CH), 3.75-3.95 (4H, m, 2×CH₂), 3.97 (1H,q, CH), 4.55(1H, m, CH), 5.34 (2H, m, CH₂), 7.31 (1H, t, CH), 7.43 (2H, t, 2×Ar-H), 7.52 (1H, t, Ar-H), 7.70 (1H, dd, Ar-H)

### Synthesis of 3-N-[2-(2-nitrophenyl)-propyloxymethyl]thymidine dimethoxytrityl ether (Compound 8)

Compound 7 (1.26 g, 2.89 mmol) and dimethoxytrityl chloride (1.19 g, 3.51 mmol) were dissolved in 20 mL of pyridine, and the obtained mixture was then stirred in a nitrogen atmosphere at room temperature overnight. Thereafter, the solvent was distilled away under reduced pressure, and the residue was then dissolved in 30 mL of ethyl acetate. The organic layer was washed with a saturated saline, and was then dried over sodium sulfate. Thereafter, the solvent was distilled away under reduced pressure. The residue was purified by silica gel chromatography using 3% triethylamine-containing hexane : ethyl acetate (1 : 1) as a developing solvent, so as to obtain 1.31 g of a product of interest (Compound 8) (1.77 mmol, yield: 61%)
¹H NMR (CDCl₃) δ=1.31 (3H, d, CH₃), 1.92 (3H,d, CH₃), 2.28-2.50 (2H, m, CH₂), 3.56 (1H, m, CH), 3.76-3.90 (10H, m, 2×CH₂, 2×CH₃), 3.92 (1H, m, CH), 4.56 (1H, m, CH), 5.37 (2H, m, CH₂), 6.16 (1H, d, CH), 6.84 (4H, d, 4×Ar-H), 7.17 (3H, d, 3×Ar-H), 7.27-7.36 (7H, m, 6×Ar-H, CH), 7.36 (1H, d, Ar-H), 7.47 (1H, d, Ar-H), 7.51 (1H, q, Ar-H), 7.71 (1H, d, Ar-H)
ESI-MS [M+Na]⁺ *m*/*z*=760.8

### Synthesis of 3-N-[2-(2-nitrophenyl)-propyloxymethyl]thymidine phosphoramidite (Compound 9)

Compound 8 (0.30 g, 0.406 mmol) was dissolved in 2 mL of dry acetonitrile in a nitrogen atmosphere. Diisopropylethylamine and 2-cyanoethyl-diisopropyl-chlorophosphoramide (200 µL, 0.912 mmol) were added to the solution, and the obtained mixture was then stirred at room temperature for 1.5 hours. Thereafter, the solvent was distilled away under reduced pressure, and a saturated sodium bicarbonate solution and ethyl acetate were then added to the residue to extract the organic layer. The organic layer was washed with a saturated saline, and was then dried over sodium sulfate. Thereafter, the solvent was distilled away under reduced pressure. The residue was purified by silica gel chromatography using 3% triethylamine-containing chloroform : methanol (20 : 1) as a developing solvent, so as to obtain 0.25 g of a product of interest (Compound 9) (0.26 mmol, yield: 65%).
ESI-MS [M+Na]⁺ *m*/*z*=961.7

### INDUSTRIAL APPLICABILITY

According to the present invention, there can be provided a method for preparing a DNA fragment, in which a desired double-stranded DNA fragment having a sticky end is directly and easily obtained from an amplification product (an amplified fragment) after PCR without a restriction enzyme digestion. In addition, according to the present invention, there can be provided a novel PCR primer that can be used in the above-described preparation method. Moreover, according to the present invention, there can be provided a genetic recombination method using the DNA fragment obtained by the above-described preparation method.

According to the preparation method of the present invention, either a DNA fragment having a sticky end consisting of a nucleotide sequence identical to that of a sticky end obtained by a restriction enzyme digestion, or a DNA fragment having a sticky end consisting of a nucleotide sequence different from that of a sticky end obtained by a restriction enzyme digestion, can be easily obtained as a double-stranded DNA fragment having a sticky end. Herein, with regard to the latter DNA fragment, the base length of a sticky end portion thereof (a single-stranded DNA portion) can be set at a desired length by arbitrarily designing the base length of a portion that does not bind to a template DNA in a PCR primer. Thus, such base length is not particularly limited. Accordingly, it is easily possible to prepare a DNA fragment whose specificity and binding efficiency have been improved to the desired levels, in terms of the binding of the DNA fragment to a host DNA. Furthermore, when a DNA fragment having a sticky end portion with a long base length is used in genetic recombination, for example, the DNA fragment is able to bind to a host DNA only by mixing them, without using a conventional DNA ligase such as a ligase. Therefore, the present invention can also provide a genetic recombination method that is excellent in terms of simplicity, economic efficiency and practical utility.

### Sequence Listing Free Text

- SEQ ID NO: 1: synthetic DNA
- SEQ ID NO: 1: t is modified with a 2-(2-nitrophenyl)propyl group (NPP group) (position 4).
- SEQ ID NO: 2: synthetic DNA
- SEQ ID NO: 3: synthetic DNA
- SEQ ID NO: 3: t is modified with a 2-(2-nitrophenyl)propyl group (NPP group) (position 3).
- SEQ ID NO: 3: t is modified with a 2-(2-nitrophenyl)propyl group (NPP group) (position 3).
- SEQ ID NO: 4: synthetic DNA
- SEQ ID NO: 4: t is modified with a 2-(2-nitrophenyl)propyl group (NPP group) (position 3).
- SEQ ID NO: 4: t is modified with a 2-(2-nitrophenyl)propyl group (NPP group) (position 4).
- SEQ ID NO: 5: synthetic DNA
- SEQ ID NO: 5: t is modified with a 2-(2-nitrophenyl)propyl group (NPP group) (position 9).
- SEQ ID NO: 5: t is modified with a 2-(2-nitrophenyl)propyl group (NPP group) (position 16).
- SEQ ID NO: 6: synthetic DNA
- SEQ ID NO: 6: t is modified with a 2-(2-nitrophenyl)propyl group (NPP group) (position 8).
- SEQ ID NO: 6: t is modified with a 2-(2-nitrophenyl)propyl group (NPP group) (position 16).
- SEQ ID NO: 7: cloning vector pBR322
- SEQ ID NO: 8: cloning vector pBR322
- SEQ ID NO: 9: cloning vector pQBI T7-GFP
- SEQ ID NO: 10: cloning vector pQBI T7-GFP
- SEQ ID NO: 11: t is modified with a 2-(2-nitrophenyl)propyl group (NPP group) (position 10).
- SEQ ID NO: 11: t is modified with a 2-(2-nitrophenyl)propyl group (NPP group) (position 10).
- SEQ ID NO: 12: t is modified with a 2-(2-nitrophenyl)propyl group (NPP group) (position 11).
- SEQ ID NO: 12: t is modified with a 2-(2-nitrophenyl)propyl group (NPP group) (position 11).
- SEQ ID NO: 13: t is modified with a 2-(2-nitrophenyl)propyl group (NPP group) (position 11).
- SEQ ID NO: 13: t is modified with a 2-(2-nitrophenyl)propyl group (NPP group) (position 11).
- SEQ ID NO: 14: t is modified with a 2-(2-nitrophenyl)propyl group (NPP group) (position 10).
- SEQ ID NO: 14: t is modified with a 2-(2-nitrophenyl)propyl group (NPP group) (position 10).
- SEQ ID NO: 15: cloning vector pQBI67 (BFP gene)

## Claims

1. A primer used for PCR, which is composed of a complementary DNA portion consisting of a nucleotide sequence complementarily binding to an amplification target region in a template DNA and a non-complementary DNA portion consisting of a nucleotide sequence that links to the 5' end of the complementary DNA portion but does not complementarily bind to the amplification target sequence,
wherein at least a base corresponding to the 3' end in the nucleotide sequence of the non-complementary DNA portion is modified with a protecting group capable of terminating the progression of DNA replication catalyzed by a DNA polymerase.

2. The primer according to claim 1, wherein the protecting group can be dissociated from the modified base by a light irradiation treatment, an alkali treatment, an acid treatment, an oxidation treatment, a reduction treatment, a desilylation treatment, a heat treatment, an esterase treatment or a phosphatase treatment.

3. The primer according to claim 2, wherein the protecting group that can be dissociated from the modified base by the light irradiation treatment is a 2-(2-nitrophenyl)propyl group, a 2-(2-nitrophenyl)propyloxymethyl group, a 1-(2-nitrophenyl)ethyl group or a 6-nitropiperonyloxymethyl group.

4. The primer according to claim 2, wherein the protecting group that can be dissociated from the modified base by the alkali treatment is an isobutyryl group, a benzoyl group or an acetoxymethyl group.

5. The primer according to claim 2, wherein the protecting group that can be dissociated from the modified base by the acid treatment is a trityl group or a methoxy derivative thereof.

6. The primer according to claim 2, wherein the protecting group that can be dissociated from the modified base by the oxidation treatment is an allyloxymethyl group, a dimethoxybenzyloxymethyl group or a trimethoxybenzyloxymethyl group.

7. The primer according to claim 2, wherein the protecting group that can be dissociated from the modified base by the reduction treatment is a benzyloxymethyl group, or a benzyloxymethyl group substituted with any given substituent.

8. The primer according to claim 2, wherein the protecting group that can be dissociated from the modified base by the desilylation treatment is a t-butyldimethoxysilyloxymethyl group or a t-butyldiphenylsilyloxymethyl group.

9. The primer according to claim 2, wherein the protecting group that can be dissociated from the modified base by the heat treatment is an isocyanate group.

10. The primer according to claim 2, wherein the protecting group that can be dissociated from the modified base by the esterase treatment is an acetoxymethyl group.

11. The primer according to claim 2, wherein the protecting group that can be dissociated from the modified base by the phosphatase treatment is a methyl phosphate group.

12. The primer according to any one of claims 3 to 11, wherein at least a base corresponding to the 3' end in the nucleotide sequence of the non-complementary DNA portion is thymine or guanine.

13. The primer according to claim 1, wherein the non-complementary DNA portion is 1 to 100 bases in length.

14. A method for preparing a DNA fragment having a sticky end, which comprises:
(i) a step of performing a PCR reaction using a template DNA and the primer according to any one of claims 1 to 13 to obtain an amplified DNA fragment; and
(ii) a step of performing a prescribed treatment on the amplified DNA fragment to dissociate a protecting group from the fragment.

15. The method according to claim 14, wherein the prescribed treatment for dissociating the protecting group from the modified base is a light irradiation treatment, an alkali treatment, an acid treatment, an oxidation treatment, a reduction treatment, a desilylation treatment, a heat treatment, an esterase treatment or a phosphatase treatment.

16. The method according to claim 14, wherein the sticky end has the same nucleotide sequence as that of a sticky end obtained by a restriction enzyme digestion.

17. The method according to claim 14, wherein the sticky end has a nucleotide sequence different from that of a sticky end obtained by a restriction enzyme digestion.

18. A genetic recombination method, which comprises a step of binding the DNA fragment obtained by the method according to any one of claims 14 to 17 to a host DNA.

19. The method according to claim 18, wherein the binding operation is carried out without using a DNA ligase.

20. A substituent-introducing agent comprising a compound represented by the following formula (I'): 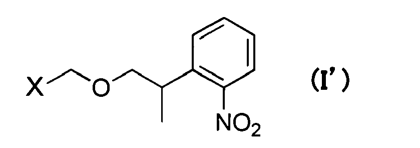 [wherein, in the formula (I'), X represents Cl, I, Br, p-toluenesulfonic acid ester or sulfuric acid ester].

21. A biomolecule having a 2-(2-nitrophenyl)propyloxymethyl group.

22. The biomolecule according to claim 21, which is represented by the following formula (I): 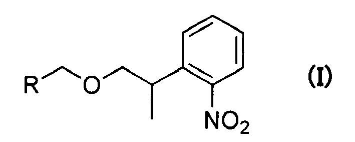 [wherein, in the formula (I), R represents a biomolecule].

23. The biomolecule according to claim 21 or 22, which is a base.

24. The biomolecule according to claim 23, wherein the base is thymine or guanine.

25. The biomolecule according to claim 24, which is represented by the following formula (II): 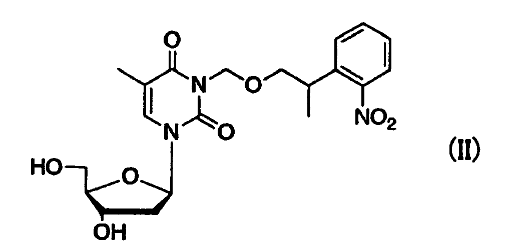 or the following formula (III):
